(19) Europäisches Patentamt
European Patent Office
Office européen des brevets

(11) Veröffentlichungsnummer : **0 166 696 B1**

# (12) EUROPÄISCHE PATENTSCHRIFT

(45) Veröffentlichungstag der Patentschrift : **19.07.89**

(21) Anmeldenummer : **85810292.4**

(22) Anmeldetag : **24.06.85**

(51) Int. Cl.[4] : **C 10 M135/20**, C 07 C149/18, C 07 C149/20, C 07 C149/267, C 07 C149/26, C 07 C149/42, C 07 C149/23, C 07 D303/34, C 07 D293/10, C 07 D293/04, C 07 C149/273

## (54) Zusätze für Schmierstoffe.

(30) Priorität : **29.06.84 CH 3148/84**
**14.05.85 CH 2047/85**

(43) Veröffentlichungstag der Anmeldung : **02.01.86 Patentblatt 86/01**

(45) Bekanntmachung des Hinweises auf die Patenterteilung : **19.07.89 Patentblatt 89/29**

(84) Benannte Vertragsstaaten : **BE DE FR GB IT**

(56) Entgegenhaltungen :
**EP-A- 0 001 217**
**DE-A- 2 730 414**
**US-A- 4 246 127**
**BULLETIN OF THE ACADEMY OF SCIENCES OF THE USSR, DIVISION OF CHEMICAL SCIENCE, Band 33, Nr. 6, Teil 2, Juni 1984, Seiten 1274-1282, Plenum Publishing Corporation, New York, US; A.R. DERZHINSKII et al.: "Functional sulfur-containing compounds. Communication 6. Synthesis and reactions of 2,3-epoxy(epithio)propyl alkyl sulfides, sulfoxides, and sulfones"**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Wirth, Hermann O., Dr.**
**Lessingstrasse 24**
**D-6140 Bensheim 3 (DE)**
Erfinder : **Friedrich, Hans-Helmut**
**Am Rauhenstein 8**
**D-6147 Lautertal 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

## Beschreibung

Die vorliegende Erfindung betrifft Schmierstoffe und Hydraulikflüssigkeiten, enthaltend thioäthergruppenhaltige Verbindungen, die Verwendung dieser Verbindungen als Additive und thioäthergruppenhaltige Verbindungen.

Aus der US-PS 4 246 127 sind Mercaptane, Thioäther, Di- und Polysulfide und deren Einsatz als Schmierstoffzusätze bekannt.

Schmierstoffen werden im allgemeinen verschiedene Zusatzstoffe zur Verbesserung ihrer Gebrauchseigenschaften beigegeben. Da Schmierstoffe zur Uebertragung grösserer Kräfte ein hohes Lasttragevermögen benötigen, werden diesen sogenannte Hochdruck- und Antiverschleiss-Additive zugesetzt, wodurch die sonst auftretenden Verschleisserscheinungen stark erniedrigt werden. Wenn andererseits z. B. Sauerstoff und Feuchtigkeit gleichzeitig auf eine Metalloberfläche einwirken, kann Korrosion auftreten, weshalb Korrosionsinhibitoren mit dem Ziel zugegeben werden, den Zutritt solcher Stoffe zur Metalloberfläche zu verhindern. Die beispielsweise bei erhöhter Temperatur verstärkt durch Luftsauerstoff eintretenden Oxidationsreaktionen in einem Schmierstoff können durch Zugabe von Antioxidantien unterbunden werden. Es ist bekannt, dass bestimmte Stoffe als Additive für Schmierstoffe eine Anzahl derartiger Eigenschaften in sich vereinigen können ; wie werden als sogenannte Vielzweck-Additive bezeichnet. Solche Stoffe sind natürlich aus ökonomischen und praktischen Gründen sehr gefragt.

Die Verbindungen dieser Erfindung vereinigen einige dieser Eigenschaften.

Die vorliegende Erfindung betrifft Zusammensetzungen, enthaltend ein Schmiermittel oder eine Hydraulikflüssigkeit und wenigstens eine Verbindung der Formel I

$$R{-}S{-}CH_2{-}\underset{\displaystyle OH}{\underset{|}{CH}}{-}CH_2{-}S{-}R^4 \qquad (I)$$

worin R ein Radikal der Form

$$R^1{-}\overset{\displaystyle R^3}{\overset{|}{\underset{\underset{\displaystyle R^2}{|}}{C}}}{-}$$

sein kann, wobei $R^1$, $R^2$ und $R^3$ unabhängig voneinander $C_1$-$C_{18}$-Alkyl sind und zusammen nicht mehr als 22 C-Atome besitzen und $R^2$ und $R^3$ ausserdem Wasserstoff sind, oder worin R $C_5$-$C_6$-Cycloalkyl, unsubstituiertes oder durch $C_1$-$C_4$-Alkyl substituiertes Phenyl oder Naphthyl, Benzyl, Furyl, Thienyl, Morpholinyl, Imidazolyl, Thiazolyl, Oxazolyl, Imidazolinyl, Thiazolinyl, Oxazolinyl, Benzimidazolinyl, Benzthiazolinyl, Benzoxazolinyl ist, und worin $R^4$ unsubstituiertes oder durch $—NH_2$ substituiertes Phenyl, unsubstituiertes oder durch Phenyl, $—NH_2$, 2-Oxopyrrolidino, Cyano, Perfluoro-$C_1$-$C_8$-Alkyl oder eine oder zwei OH-Gruppen substituiertes $C_1$-$C_{16}$-Alkyl, das gegebenenfalls durch —O— oder —S— unterbrochen sein kann, oder $C_5$-$C_6$-Cycloalkyl ist oder $R^4$ $—(CH_2)_m—S—CH_2—CH(OH)—CH_2—S—(C_1$-$C_{16}$-Alkyl) mit m gleich 0 bis 6 ist, oder $R^4$ $—(CH_2)_n—C(O)—O—R^5$ ist, wobei n gleich 1 oder 2 und $R^5$ Wasserstoff, $C_1$-$C_{16}$-Alkyl oder Alkalimetall ist, oder worin $R^4$ $—CH[—CO—OR^5]$ $[—CH_2—CO—OR^5]$ bedeutet, wobei $R^5$ die oben gegebene Bedeutung aufweist, oder worin $R^4$ $—(CH_2)_r$-$C(O)—OH{\cdot}H_2N—(C_8$-$C_{16}$-Alkyl oder $—I(CH_2)_r$-$C(O)—OH{\cdot}N(CH_2—CH_2—OH)_3$ mit r gleich 1 oder 2 oder $—P(X)—[O—R^6]_2$ darstellt, wobei X=O oder S sein kann, und $R^6$ $C_1$-$C_{16}$-Alkyl, Phenyl oder Tolyl ist, oder worin $R^4$ α- oder β-Naphthyl, Benzthiazolyl, Benzimidazolyl, Benzoxazolyl, Thiazolyl, Thiazolinyl, Triazolyl, Tetrazolyl, Pyridyl, Chinolyl, Imidazolyl, Imidazolinyl, Oxazolinyl, $—SO_2—O—$(Alkalimetall), $—C_6H_4—C(O)—O—$(Alkalimetall), 2-Oxo-4-hydroxy-3-penten-3-yl oder $—(CH_2)_s—R^7$ ist, wobei s gleich 1 bis 4 ist und $R^7$ Benzoxazolyl, Benzimidazolyl, Benzthiazolyl, Thiazolinyl, Imidazolinyl oder Oxazolinyl darstellt, oder worin $R^4$ $—(CH_2)_t—CO—N(R^8)$ $(R^9)$ bedeutet, worin t gleich 1 oder 2 ist und $R^8$ $C_1$-$C_{16}$-Alkyl, das gegebenenfalls durch —OH substituiert sein kann, Phenyl, Hydroxyphenyl oder α-Naphthyl und $R^9$ Wasserstoff oder $R^8$ ist, oder worin $R^4$ $—CH_2—CH(OH)—CH_2—S—R^{10}$ ist, wobei $R^{10}$ Wasserstoff oder $C_1$-$C_{16}$-Alkyl ist, oder worin $R^4$ einen Rest $—R^{11}—S—CH_2—CH(OH)—CH_2—S—R$ darstellt, wobei R die oben gegebene Bedeutung hat und $R^{11}$ ein Radikal $—(CH_2)_2—O—(CH_2)_2—O—(CH_2)_2—$, o- oder m-Phenylen, Thiadiazol-2,5-ylen oder $—(CH_2)_u—$ mit u gleich 0 bis 8 ist oder ein Radikal der Formeln

(Siehe Formeln Seite 3 f.)

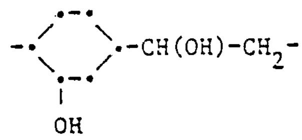 oder

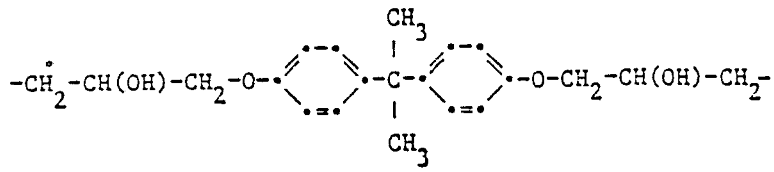

darstellt.

Stellt R ein Radikal der Form

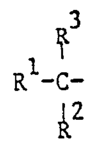

dar, so kann es sich um $R^1-CH_2-$,

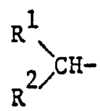 oder um 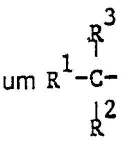

handeln, wobei $R^1$, $R^2$ und $R^3$ jeweils $C_1$-$C_{18}$-Alkyl sind. Bei $C_1$-$C_{18}$-Alkyl handelt es sich um geradkettige oder verzweigte Substituenten, wie beispielsweise Methyl, Ethyl, n-Propyl, Isopropyl, n-Butyl, Isobutyl, sec.-Butyl, tert.-Butyl, geradkettiges oder verzweigtes Pentyl, Hexyl, Heptyl, Octyl, Nonyl, Decyl, Undecyl, Dodecyl, Tridecyl, Tetradecyl, Pentadecyl, Hexadecyl, Heptadecyl oder Octadecyl. Bevorzugt ist

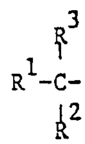

worin $R^1$, $R^2$ und $R^3$ zusammen mit dem C-Atom, an das sie gebunden sind, $C_4$-$C_{20}$-Alkyl bilden, wobei keiner dieser Substituenten $R^1$, $R^2$ und $R^3$ Wasserstoff sein darf ; besonders bevorzugt ist hier nun $C_4$-$C_{14}$-Alkyl, insbesondere ist tert.-Butyl, tert.-Nonyl (ex Phillips Petroleum) oder tert.-Dodecyl bevorzugt, wobei z. B. unter tert.-Dodecyl solch ein Rest verstanden werden soll, wie er für tertiäres Dodecylmercaptan in « Ullmanns Enzyklopädie der technischen Chemie, 4. Auflage, Band 23, Seite 181-182, Verlag Chemie, Weinheim » beschrieben ist.

Stellt R $C_5$-$C_6$-Cycloalkyl dar, so handelt es sich um Cyclopentyl oder Cyclohexyl.

Stellt R durch $C_1$-$C_4$-Alkyl substituiertes Phenyl oder Naphthyl dar, so können Phenyl oder Naphthyl ein- bis dreifach, bevorzugt jedoch einfach, substituiert sein ; bei $C_1$-$C_4$-Alkyl handelt es sich um Methyl, Ethyl, n-Propyl, Isopropyl, n-Butyl, Isobutyl, sec.-Butyl oder tert.-Butyl.

Ist $R^4$ $C_1$-$C_6$-Alkyl oder liegt in den Substituenten für $R^4$, wie bei $-(CH_2)_m-S-CH_2-CH(OH)-S-(C_1\text{-}C_{16}\text{-Alkyl})$, $-(CH_2)_n-C(O)-O-(C_1\text{-}C_{16}\text{-Alkyl})$, $-P(X)-[O-R^6]_2$ oder $-CH_2-CH(OH)-CH_2-S-(C_1\text{-}C_{16}\text{-Alkyl})$, oder $R^5$, $R^8$ oder $R^{10}$ ein $C_1$-$C_{16}$-Alkylrest vor, so handelt es sich um geradkettige oder verzweigte Alkylradikale, wie beispielsweise Methyl, Ethyl, n-Propyl, Isopropyl, n-Butyl, Isobutyl, sec.-Butyl, tert.-Butyl, oder geradkettiges oder verzweigtes Pentyl, Hexyl, Heptyl, Octyl, Nonyl, Decyl, Undecyl, Dodecyl, Tridecyl, Tetradecyl, Pentadecyl oder Hexadecyl, bevorzugt sind Isopropyl, tert.-Butyl, Isooctyl, 2-Ethylhexyl, tert.-Nonyl, tert.-Dodecyl oder tert.-Tridecyl. Dabei wird unter Isooctyl ein Rest verstanden, der sich vom Isooctylalkohol ableitet, und eine Mischung verschieden verzweigter Octylreste ist ; für tert.-Nonyl sowie für tert.-Dodecyl sind die oben bereits gegebenen Definitionen anzuwenden.

Liegt für $R^4$ $-(CH_2)_r-C(O)-OH \cdot H_2N-(C_8\text{-}C_{16}\text{-Alkyl})$ vor, so handelt es sich darin bei $C_8$-$C_{16}$-Alkyl um geradkettige oder verzweigte Substituenten, wie beispielsweise Octyl, Nonyl, Decyl, Undecyl, Dodecyl, Tridecyl, Tetradecyl, Pentadecyl oder Hexadecyl, bevorzugt um tert.-Tridecyl. Als Amin kommt ferner in Frage $N-(CH_2-CH_2-OH)_3$.

Ist $R^4$ durch Phenyl substituiertes $C_1$-$C_{16}$-Alkyl, so handelt es sich bevorzugt um durch Phenyl substituiertes $C_1$-$C_4$-Alkyl, wobei das Phenyl endständig ist, besonders bevorzugt um Benzyl.

Ist $R^4$ durch ein oder zwei OH-Gruppen substituiertes $C_1$-$C_{16}$-Alkyl, so handelt es sich bevorzugt um $-CH_2-CH_2-OH$, $-CH(OH)-CH_2-OH$ oder $-CH_2-CH(OH)-CH_2-OH$.

Ist $R^4$ durch $-NH_2$ substituiertes $C_1$-$C_{16}$-Alkyl, so handelt es sich bevorzugt um $-CH_2-CH_2-NH_2$.

Ist $R^5$ Alkalimetall oder tritt letzteres in $-SO_2-O-$(Alkalimetall) oder $-C_6H_4-CO-O-$ (Alkalimetall) auf, so handelt es sich bevorzugt um Natrium oder Kalium.

Bevorzugt sind Zusammensetzungen, enthaltend ein Schmiermittel oder eine Hydraulikflüssigkeit und wenigstens eine Verbindung der Formel II

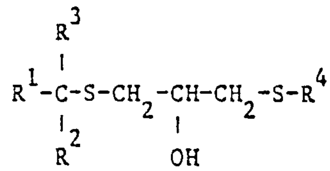

(II)

worin $R^1$, $R^2$ und $R^3$ zusammen mit dem C-Atom, an das sie gebunden sind, $C_4$-$C_{20}$-Alkyl sind, wobei keiner dieser Substituenten $R^1$, $R^2$ und $R^3$ Wasserstoff sein darf, und worin $R^4$ unsubstituiertes oder durch $-NH_2$ substituiertes Phenyl, unsubstituiertes oder durch Phenyl, $-NH_2$, 2-Oxo-pyrrolidino, Cyano, Perfluoro-$C_1$-$C_8$-Alkyl, oder eine oder zwei OH-Gruppen substituiertes $C_1$-$C_{16}$-Alkyl, das gegebenenfalls durch $-O-$ oder $-S-$ unterbrochen sein kann, oder $C_5$-$C_6$-Cycloalkyl ist oder $R^4$ $-(CH_2)_m-S-CH_2-CH(OH)-CH_2-S-$($C_1$-$C_{16}$-Alkyl) mit m gleich 0 bis 6 oder $-(CH_2)_n-C(O)-O-R^5$ ist, wobei n gleich 1 oder 2 und $R^5$ Wasserstoff, $C_1$-$C_{16}$-Alkyl oder Alkalimetall ist, oder worin $R^4$ $-CH[-CO-OR^5][-CH_2-CO-OR^5]$ bedeutet, wobei $R^5$ die oben gegebene Bedeutung aufweist, oder worin $R^4$ $-(CH_2)_r-C(O)-OH\cdot H_2N-$($C_8$-$C_{16}$-Alkyl) oder $-(CH_2)_r-C(O)-OH\cdot N-(CH_2-CH_2-OH)$ mit r gleich 1 oder 2, oder $-P(X)-[O-R^6]_2$ darstellt, wobei X=O oder =S sein kann und $R^6$ $C_1$-$C_{16}$-Alkyl, Phenyl oder Tolyl ist, oder worin $R^4$ α- oder β-Naphthyl, Benzthiazolyl, Benzimidazolyl, Benzoxazolyl, Thiazolyl, Thiazolinyl, Pyridyl, Chinolyl, Imidazolinyl, Oxazolinyl, $-SO_2-O-$(Alkalimetall), $C_6H_4-C(O)-O-$(Alkalimetall), 2-Oxo-4-hydroxy-3-penten-3-yl, $-C_6H_4-C(O)-O-$(Alkalimetall), 2-Oxo-4-hydroxy-3-penten-3-yl, $-(CH_2)_s-R^7$ ist, wobei s gleich 1 bis 4 ist und $R^7$ Benzoxazolyl, Benzimidazolyl, Benzthiazolyl, Thiazolinyl, Imidazolinyl oder Oxazolinyl darstellt, oder worin $R^4$ $-(CH_2)_t-CO-N(R^8)(R^9)$ bedeutet, wobei t gleich 1 oder 2 ist und $R^8$ $C_1$-$C_{16}$-Alkyl, das gegebenenfalls durch $-OH$ substituiert sein kann, Phenyl, 3-Hydroxyphenyl oder α-Naphthyl und $R^9$ Wasserstoff oder $R^8$ ist, oder worin $R^4$ $-CH_2-CH(OH)-CH_2-S-R^{10}$ ist, wobei $R^{10}$ Wasserstoff oder $C_1$-$C_{16}$-Alkyl ist, oder worin $R^4$ einen Rest

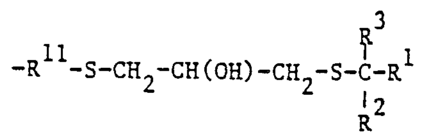

darstellt, wobei $R^1$, $R^2$ und $R^3$ die oben gegebene Bedeutung haben und $R^{11}$ ein Radikal $-(CH_2)_2-O-(CH_2)_2-O-(CH_2)_2-$, o- oder m-Phenylen, Thiadiazol-2,5-ylen oder $-(CH_2)_u-$ mit u gleich 0 bis 8, bevorzugt 2, ist oder ein Radikal der Formeln

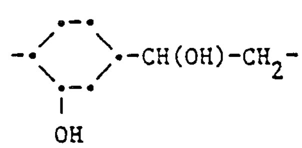

oder

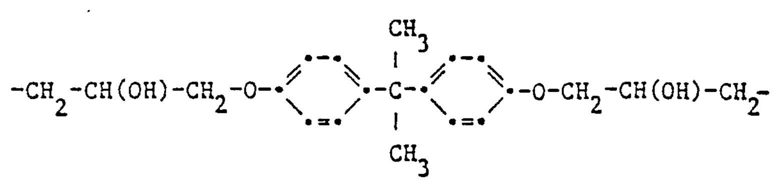

darstellt.

Besonders bevorzugt sind Zusammensetzungen, enthaltend ein Schmiermittel oder eine Hydraulikflüssigkeit und wenigstens eine Verbindung der Formel II, worin $R^1$, $R^2$ und $R^3$ zusammen mit dem C-Atom, an das sie gebunden sind, $C_4$-$C_{20}$-Alkyl sind, wobei keiner dieser Substituenten $R^1$, $R^2$ und $R^3$ Wasserstoff sein darf, und worin $R^4$ unsubstituiertes oder durch $-NH_2$ substituiertes Phenyl, unsubstituiertes oder durch Phenyl, $-NH_2$, 2-Oxopyrrolidino, Cyano oder eine oder zwei OH-Gruppen substituier-

tes $C_1$-$C_{13}$-Alkyl, das gegebenenfalls durch —O— oder —S— unterbrochen sein kann, ist oder worin $R^4$ $-(CH_2)_m-S-CH_2-CH(OH)-CH_2-S-(C_1\text{-}C_{16})$-Alkyl) mit m gleich 0 bis 4, oder $-(CH_2)_n-CO-O-R^5$ ist, wobei $R^5$ Wasserstoff, Kalium oder $C_4$-$C_{12}$-Alkyl und n gleich 1 oder 2 ist, oder worin $R^4$ $-(CH_2)_r-CO-OH\cdot H_2N-(C_{10}\text{-}C_{16}$-Alkyl) mit r gleich 1 oder 2, $-P(S)-[O-R^6]_2$ darstellt, und $R^6$ $C_1$-$C_8$-Alkyl oder Phenyl ist, oder worin $R^4$ α-Naphthyl, Thiazolyl, Benzthiazolyl, Benzimidazolyl, Benzoxazolyl, Pyridyl, Chinolyl oder $-(CH_2)_s-R^7$ ist, wobei s gleich 1 oder 2 ist und $R^7$ Benzoxazolyl, Benzimidazolyl, Benzthiazolyl, Thiazolinyl, Imidazolinyl oder Oxazolinyl ist, oder worin $R^4$ $-(CH_2)_t-CO-N(R^8)$ $(R^9)$ ist, wobei t gleich 1 oder 2 ist und $R^8$ $C_1$-$C_4$-Alkyl, das gegebenenfalls durch —OH substituiert sein kann, Phenyl oder α-Naphthyl ist, und $R^9$ Wasserstoff oder $R^8$ ist, oder worin $R^4$ $-CH_2-CH(OH)-CH_2-S-R^{10}$ ist, wobei $R^{10}$ $C_1$-$C_{14}$-Alkyl ist, oder worin $R^4$ einen Rest

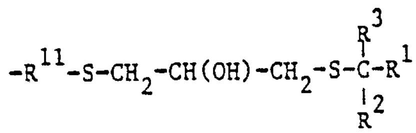

darstellt, wobei $R^1$, $R^2$ und $R^3$ die oben gegebene Bedeutung haben und $R^{11}$ $-(CH_2)_2-O-(CH_2)_2-O-(CH_2)_2-$, o- oder m-Phenylen, Thiadiazol-2,5-ylen oder $-(CH_2)_u-$ mit u gleich 0 bis 4, bevorzugt 2, ist.

Insbesondere bevorzugt sind Zusammensetzungen, enthaltend ein Schmiermittel oder eine Hydraulikflüssigkeit und wenigstens eine Verbindung der Formel II, worin $R^1$, $R^2$ und $R^3$ zusammen mit dem C-Atom, an das sie gebunden sind, $C_4$-$C_{14}$-Alkyl sind, wobei keiner dieser Substituenten $R^1$, $R^2$ und $R^3$ Wasserstoff sein darf, und worin $R^4$ Phenyl, $-CH_2-CH_2-NH_2$, $-CH_2-CH_2-OH$, $-CH_2-CH(OH)-CH_2-OH$, tertiäres $C_4$-$C_{14}$-Alkyl, $-(CH_2)_2-S-CH(OH)-CH_2-S-$(tert.-$C_8$-$C_{12}$-Alkyl), $-CH_2-COOH$ $-CH_2-CO-O-(i\text{-}C_8H_{17})$, $-CH_2-CO-OH\cdot H_2N-$(tert.-$C_{10}$-$C_{16}$-Akyl), $-P(S)-[O\text{-}(i\text{-}C_3H_7)]_2$, $-P(S)-[O\text{-}(i\text{-}C_8H_{17})]_2$, α-Naphthyl, Benzthiazolyl, Benzimidazolyl, Thiazolyl oder $-CH_2-CH(OH)-CH_2-S-R^{10}$ ist, wobei $R^{10}$ tertiäres $C_4$-$C_{14}$-Alkyl ist, oder worin $R^4$ einen Rest

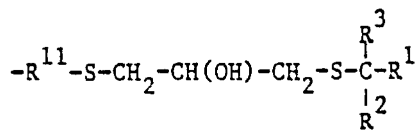

darstellt, wobei $R^1$, $R^2$ und $R^3$ die oben gegebene Bedeutung haben und $R^{11}$ $-(CH_2)_2-O-(CH_2)_2-O-(CH_2)_2-$, o-Phenylen, Thiadiazol-2,5-ylen oder $-(CH_2)_u-$ mit u gleich 0 bis 2 ist.

Einen weiteren Gegenstand der Erfindung bilden neue Stoffe der Formel II, worin $R^1$, $R^2$ und $R^3$ zusammen mit dem C-Atom, an das sie gebunden sind, $C_4$-$C_{20}$-Alkyl sind, wobei keiner dieser Substituenten $R^1$, $R^2$ und $R^3$ Wasserstoff sein darf, und worin $R^4$ unsubstituiertes oder durch $-NH_2$ substituiertes Phenyl, unsubstituiertes $C_1$-$C_9$-Alkyl oder durch Phenyl, $-NH_2$, 2-Oxopyrrolidino, Cyano, Perfluoro-$C_1$-$C_8$-Alkyl oder eine oder zwei OH-Gruppen substituiertes $C_1$-$C_{16}$-Alkyl ist, das gegebenenfalls durch —O— oder —S— unterbrochen sein kann, oder worin $R^4$ $-(CH_2)_m-S-CH_2-CH(OH)-CH_2-S-(C_1\text{-}C_{16}$-Alkyl) mit m gleich 0 bis 6, oder $-(CH_2)_n-C(O)-O-R^5$ ist, wobei n gleich 1 oder 2 und $R^5$ Wasserstoff, $C_1$-$C_{16}$-Alkyl oder Alkalimetall ist, oder worin $R^4$ $-CH[-CO-OR^5]$ $[-CH_2-CO-OR^5]$ bedeutet, wobei $R^5$ die oben gegebene Bedeutung aufweist, oder worin $R^4$ $-(CH_2)_r-C(O)-OH\cdot H_2N-(C_8\text{-}C_{16}$-Alkyl) oder $-(CH_2)_r-C(O)-OH\cdot N(-CH_2-CH_2-OH)_3$ mit r gleich 1 oder 2, oder $-P(X)-[O-R^6]_2$ darstellt, wobei X=O oder =S sein kann, und $R^6$ $C_1$-$C_{16}$-Alkyl, Phenyl oder Tolyl ist, oder worin $R^4$ α- oder β-Naphthyl, Benzthiazolyl, Benzimidazolyl, Benzoxazolyl, Thiazolyl, Thiazolinyl, Pyridyl, Chinolyl, Imidazolinyl, Oxazolinyl, $-SO_2-O-$(Alkalimetall), $-C_6H_4-C(O)-O-$(Alkalimetall), 2-Oxo-4-hydroxy-3-penten-3-yl, oder $-(CH_2)_s-R$ ist, wobei s gleich 1 bis 4 ist und $R^7$ Benzoxazolyl, Benzimidazolyl, Benzthiazolyl, Thiazolinyl, Imidazolinyl oder Oxazolinyl darstellt, oder worin $R^4$ $-(CH_2)_t-CO-N(R^8)$ $(R^9)$ bedeutet wobei t gleich 1 oder 2 ist und $R^8$ $C_1$-$C_{16}$-Alkyl, das gegebenenfalls durch —OH substituiert sein kann, Phenyl, 3-Hydroxyphenyl oder α-Naphthyl ist und $R^9$ Wasserstoff oder $R^8$ ist, oder worin $R^4$ $-CH_2-CH(OH)-CH_2-S-R^{10}$ ist, wobei $R^{10}$ Wasserstoff oder $C_1$-$C_{16}$-Alkyl ist, oder worin $R^4$ einen Rest

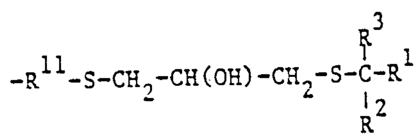

darstellt, wobei $R^1$, $R^3$ und $R^3$ die oben angegebene Bedeutung haben und $R^{11}$ ein Radikal $-(CH_2)_2-O-(CH_2)_2-O-(CH_2)_2-$, o- oder m-Phenylen, Thiadiazol-2,5-ylen oder $-(CH_2)_u-$ mit u gleich 0 bis 8 ist oder ein Radikal der Formeln

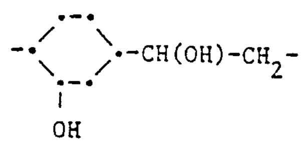

oder

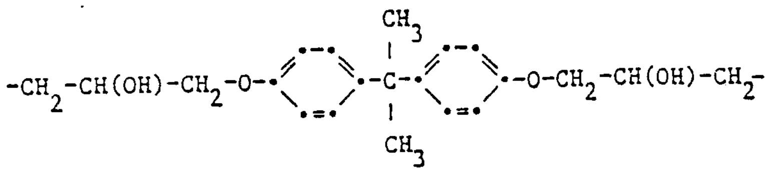

darstellt.

Bevorzugt sind Verbindungen der Formel II, worin $R^1$, $R^2$ und $R^3$ zusammen mit dem C-Atom, an das sie gebunden sind, $C_4$-$C_{20}$-Alkyl sind, wobei keiner dieser Substituenten $R^1$, $R^2$ und $R^3$ Wasserstoff sein darf, und worin $R^4$ unsubstituiertes oder durch $-NH_2$ substituiertes Phenyl, unsubstituiertes $C_4$-$C_9$-Alkyl oder durch Phenyl, $-NH_2$, 2-Oxopyrrolidino, Cyano oder eine oder zwei OH-Gruppen substituiertes $C_1$-$C_{13}$-Alkyl, $-(CH_2)_m-S-CH_2-CH(OH)-CH_2-S-(C_1\text{-}C_{16}\text{-Alkyl})$ mit m gleich 0 bis 4, oder $-(CH_2)_n-CO-O-R^5$ ist, wobei $R^5$ Wasserstoff, Kalium oder $C_4$-$C_{12}$-Alkyl und n gleich 1 oder 2 ist, oder worin $R^4$ $-(CH_2)_r-CO-OH\cdot H_2N(C_{10}\text{-}C_{16}\text{-Alkyl})$ mit r gleich 1 oder 2, $-P(S)-[O-R^6]_2$, wobei $R^6$ $C_1$-$C_8$-Alkyl oder Phenyl ist, oder worin $R^4$ α-Naphthyl, Thiazolyl, Benzthiazolyl, Benzimidazolyl, Benzoxazolyl, oder $-(CH_2)_s-R^7$ ist, wobei s gleich 1 oder 2 ist und $R^7$ Benzoxazolyl, Benzimidazolyl, Benzthiazolyl, Thiazolinyl, Imidazolinyl oder Oxazolinyl ist, oder worin $R^4$ $-(CH_2)_t-CO-N(R^8)(R^9)$ ist, wobei t gleich 1 oder 2 ist und $R^8$ $C_1$-$C_4$-Alkyl, das gegebenenfalls durch $-OH$ substituiert sein kann, Phenyl oder α-Naphthyl ist und $R^9$ Wasserstoff oder $R^8$ ist, oder worin $R^4$ $-CH_2-CH(OH)-CH_2-S-R^{10}$ ist, wobei $R^{10}$ $C_1$-$C_{14}$-Alkyl ist, oder worin $R_4$ einen Rest

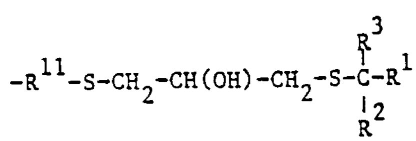

darstellt, wobei $R^1$, $R^2$ und $R^3$ die oben gegebene Bedeutung haben und $R^{11}$ $-(CH_2)_2-O-(CH_2)_2-O-(CH_2)_2-$, o- oder m-Phenylen, Thiadiazol-2,5-ylen oder $-(CH_2)_u-$ mit u gleich 0 bis 4, bevorzugt 2, ist.

Besonders bevorzugt sind Verbindungen der Formel II, worin $R^1$, $R^2$ und $R^3$ zusammen mit dem C-Atom, an das sie gebunden sind, $C_4$-$C_{14}$-Alkyl sind, wobei keiner dieser Substituenten $R^1$, $R^2$ und $R^3$ Wasserstoff sein darf, und worin $R^4$ Phenyl, $-CH_2-CH_2-NH_2$, $-CH_2-CH_2-OH$, $-CH_2-CH(OH)-CH_2-OH$, tertiäres $C_4$-$C_9$-Alkyl, $-(CH_2)_2-S-CH(OH)-CH_2-S-(\text{tert.-}C_8\text{-}C_{12}\text{-Alkyl})$, $-CH_2COOH$, $-CH_2-CO-O-(i\text{-}C_8H_{17})$, $-CH_2-CO-OH\cdot H_2N-(\text{tert.-}C_{10}\text{-}C_{16}\text{-Alkyl})$, $-P(S)-[O\text{-}(i\text{-}C_3H_7)]_2$, $-P(S)-[O\text{-}(iC_8H_{17})]_2$, α-Naphthyl, Benzthiazolyl, Benzimidazolyl, Thiazolyl oder $-CH_2-CH(OH)-CH_2-S-R^{10}$ ist, wobei $R^{10}$ tertiäres $C_4$-$C_{14}$-Alkyl ist, oder worin $R^4$ einen Rest

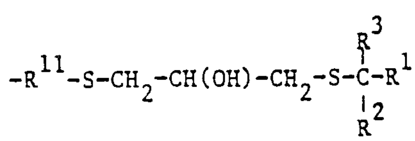

darstellt, wobei $R^1$, $R^2$ und $R^3$ die oben gegebene Bedeutung haben und $R^{11}$ $-(CH_2)_2-O-(CH_2)_2-O-(CH_2)_2-$, o-Phenylen, Thiadiazol-2,5-ylen oder $-(CH_2)_u-$ mit u gleich 0 bis 2 ist.

Beispiele für Verbindungen der Formel I sind die folgenden :

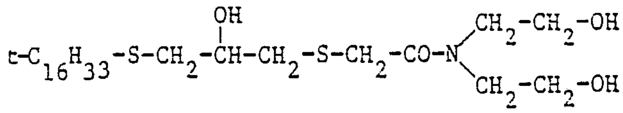

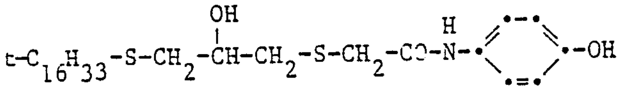

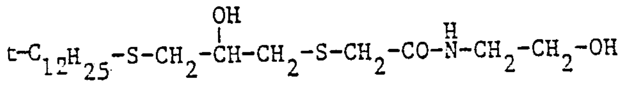

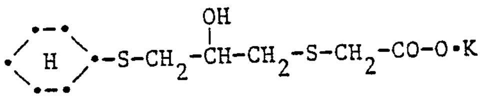

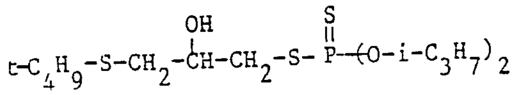

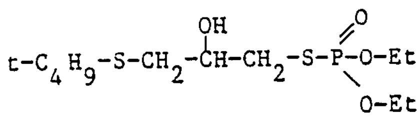

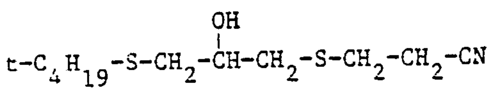

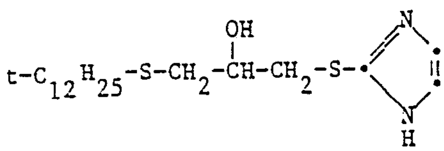

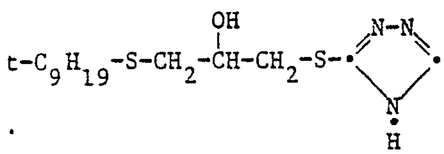

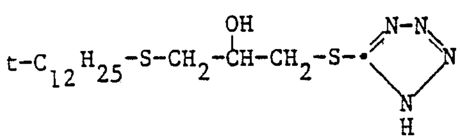

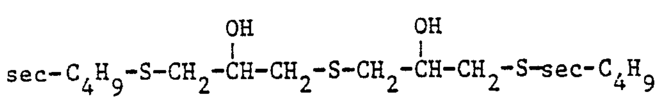

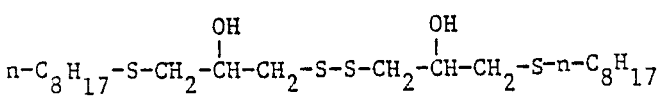

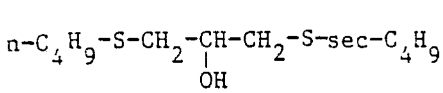

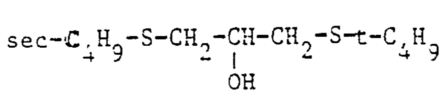

Die Herstellung der als Zwischenprodukt für die Verbindungen der Formel I fungierenden Alkylthiaglycidyläther geschieht auf folgende Art und Weise :

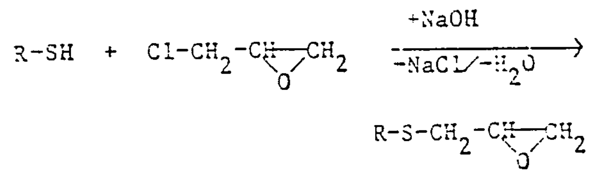


(III)

wobei der Substituent R die bereits gegebene Bedeutung hat. Besonders vorteilhaft für diese Umsetzung ist die Verwendung eines Phasentransfer-Katalysators, wie z. B. Tetrabutylaminchlorid. Die Herstellung von Alkyl-thiaglycidylethern ist auch in US 2 965 652, US 2 731 437 und BE 609 375 beschrieben.

Durch Umsetzung von Alkyl-thiaglycidyläthern der Formel III mit Verbindung der Formel IV

$$HS—R^4 \qquad (IV)$$

unter Verwendung von katalytischen Mengen von Nukleophilen, wie z. B. Natriumhydrid oder Triäthylamin, können die Verbindungen der Formel I hergestellt werden, wobei die Substituenten R und $R^4$ die bereits gegebene Bedeutung haben.

Andere Methoden, die sich zur Herstellung von Verbindungen der Formel I eignen, sind beispielsweise in DE-OS 2 730 414, aufgeführt.

Ein anderer Syntheseweg koppelt die Glycidylthioäthersynthese mit der Additionsreaktion des gleichen Mercaptans :

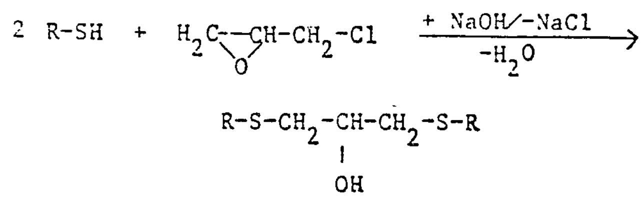


wobei ein Teil der unter die Formel I fallenden Verbindungen hergestellt werden kann.

Ein weiterer Syntheseweg führt über die folgende Reaktion

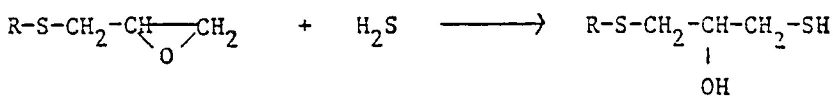

zu α-Hydroxymercaptanderivaten, die mit Chlorverbindungen Cl-$R^4$ die gewünschten Verbindungen der Formel I liefern, wobei der abgespaltene Chlorwasserstoff durch eine anorganische oder organische Base gebunden wird. Die oxidative Kupplung der α-Hydroxymercaptanderivate führt ebenfalls zu einigen Verbindungen der Formel I mit Disulfidstruktur.

Schliesslich kann auch für einen Teil der Verbindungen der Formel I der folgende Syntheseweg beschritten werden

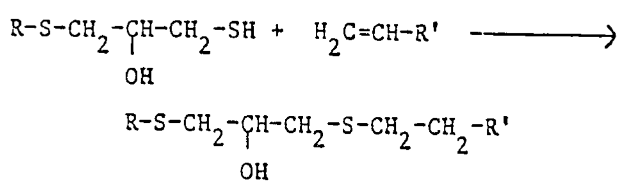

wobei $—CH_2—CH_2—R'$ ein spezielles zu $R^4$ gehöriges Radikal sein kann. Schliesslich kann eine solche α-Hydroxymercaptanverbindung mit einem Glycidyläther umgesetzt werden, die für ein Beispiel formuliert :

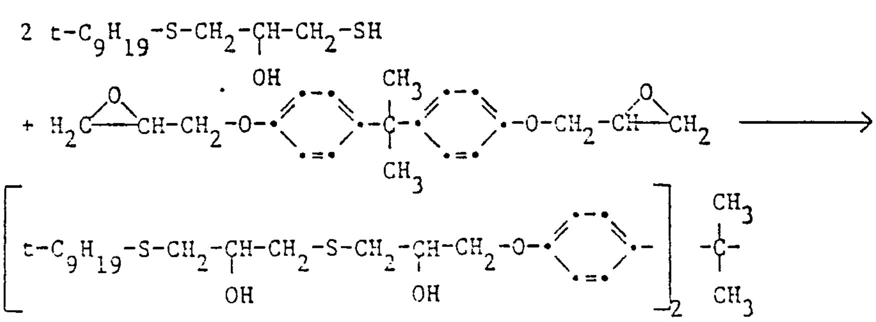

Die Verbindungen der Formel I sind von leichtflüssiger, viskoser bis wachsartiger Beschaffenheit und überraschend gut in Schmierstoffen löslich. Sie sind als Zusätze zu Schmierstoffen besonders geeignet und führen zu einer Verbesserung der Hochdruck- und Antiverschleiss-Eigenschaften, ebenso ist auch auf ihre antioxidierende und antikorrosive Wirkung hinzuweisen. Ueberraschend ist schliesslich die Herstellung von sogenannten Masterbatches möglich.

Die Verbindungen der Formel I wirken schon in sehr geringen Mengen als Additive in Schmierstoffen. Sie werden den Schmierstoffen in einer Menge von 0,01 bis 5 Gew.-%, vorzugsweise in einer Menge von 0,05 bis 3 Gew.-%, bezogen auf den Schmierstoff, zugesetzt. Die in Frage kommenden Schmierstoffe sind dem Fachmann geläufig und z. B. in « Schmierstoffe und verwandte Produkte » (Verlag Chemie, Weinheim, 1982) beschrieben. Besonders geeignet sind neben Mineralölen z. B. Poly-α-Olefine, Schmierstoffe auf Esterbasis, Phosphate, Glykole, Polyglykole und Polyalkylenglykole.

Die Schmierstoffe können zusätzlich andere Additive enthalten, die zugegeben werden, um die Grundeigenschaften von Schmierstoffen noch weiter zu verbessern ; dazu gehören : Antioxidantien, Metallpassivatoren, Rostinhibitoren, Viskositätsindex-Verbesserer, Stockpunkterniedriger, Dispergiermittel, Detergentien, Hochdruck-Zusätze und Antiverschleiss-Additive.

Beispiele für phenolische Antioxidantien

1. Alkylierte Monophenole

2,6-Di-tert-butyl-4-methylphenol
2,6-Di-tert-butylphenol
2-tert-butyl-4,6-Dimethylphenol
2,6-Di-tert-butyl-4-ethylphenol
2,6-Di-tert-butyl-4-ethylphenol
2,6-Di-tert-butyl-4-n-butylphenol
2,6-Di-tert-butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert-butyl-4-methoxymethylphenyl
o-tert-Butylphenol

2. Alkylierte Hydrochinone

2,6-Di-tert-butyl-4-methoxyphenol
2,5-Di-tert-butyl-hydrochinon
2,5-Di-tert-amyl-hydrochinon
2,6-Diphenyl-4-octadecyloxyphenol

3. Hydroxylierte Thiodiphenylether

2,2'-Thio-bis-(6-tert-butyl-4-methylphenol)
2,2'-Thio-bis-(4-octylphenol)
4,4'-Thio-bis-(6-tert-butyl-3-methylphenol)
4,4'-Thio-bis-(6-tert-butyl-2-methylphenol)

4. Alkyliden-Bisphenole

2,2'-Methylen-bis-(6-tert-butyl-4-methylphenol)
2,2'-Methylen-bis-(6-tert-butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert-butylphenol)
2,2'-Ethyliden-bis-(4,6-di-tert-butylphenol)
2,2'-Ethyliden-bis-(6-tert-butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert-butylphenol)
4,4'-Methylen-bis-(6-tert-butyl-2-methylphenol)
1,1-Bis-(5-tert-butyl-4-hydroxy-2-methylphenyl)-butan
2,6-Di-(3-tert-butyl-5-methyl-2-hydroxybenzyl)-4-methylphenol
1,1,3-Tris-(5-tert-butyl-4-hydroxy-2-methylphenyl)-3-n-dodecylmercaptobutan

Ethylenglycol-bis-[3,3-bis-(3'-tert-butyl-4'-hydroxyphenyl)-butyrat]
Di-(3-tert-butyl-4-hydroxy-5-methylphenyl)-dicyclopentadien
Di-[2-(3'-tert-butyl-2'-hydroxy-5'-methyl-benzyl)-6-tert-butyl-4-methyl-phenyl]-terephthalat.

5. Benzylverbindungen

1,3,5-Tri-(3,5-di-tert-butyl-4-hydroxybenzyl)-2,4,6-trimethylbenzol
Di-(3,5-di-tert-butyl-4-hydroxybenzyl)-sulfid
3,5-Di-tert-butyl-4-hydroxybenzyl-mercaptoessigsäure-isooctylester
Bis-(4-tert-butyl-3-hydroxy-2,6-dimethylbenzyl)-dithiol-terephthalat
1,3,5-Tris-(3,5-di-tert-butyl-4-hydroxybenzyl)-isocyanurat
1,3,5-Tris-(4-tert-butyl-3-hydroxy-2,6-dimethylbenzyl)-isocyanurat
3,5-Di-tert-butyl-4-hydroxybenzyl-phosphonsäure-dioctadecylester
3,5-Di-tert-butyl-4-hydroxybenzyl-phosphonsäure-monoethylester Calcium-salz.

6. Acylaminophenole

4-Hydroxy-laurinsäureanilid
4-Hydroxy-stearinsäureanilid
2,4-Bis-octylmercapto-6-(3,5-di-tert-butyl-4-hydroxyanilino)-s-triazin
N-(3,5-di-tert-butyl-4-hydroxyphenyl)-carbaminsäureoctylester.

7. Ester der β-(3,5-Di-tert-butyl-4-hydroxyphenyl)-propionsäure

mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

8. Ester der β-(5-tert-butyl-4-hydroxy-3-methylphenyl)-propionsäure

mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

9. Amide der β-(3,5-Di-tert-butyl-4-hydroxyphenyl)-propionsäure,

wie z. B.
N,N'-Di-(3,5-di-tert-butyl-4-hydroxyphenylpropionyl)-hexamethylendiamin
N,N'-Di-(3,5-di-tert-butyl-4-hydroxyphenylpropionyl)-trimethylendiamin
N,N'-Di-(3,5-di-tert-butyl-4-hydroxyphenylpropionyl)-hydrazin.

Beispiele für aminische Antioxidantien :

N,N'-Di-isopropyl-p-phenylendiamin
N,N'-Di-sec-butyl-p-phenylendiamin
N,N'-Bis(1,4-dimethyl-pentyl)-p-phenylendiamin
N,N'-Bis(1-ethyl-3-methyl-pentyl)-p-phenylendiamin
N,N'-Bis(1-methyl-heptyl)-phenylendiamin
N,N'-Diphenyl-p-phenylendiamin
N,N'-Di-(naphthyl-2)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec-butyl-p-phenylendiamin
Diphenylamin
4-Isopropoxy-diphenylamin
N-Phenyl-1-naphthylamin

N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert-butyl-4-dimethylamino-methyl-phenol
2,4'-Diamino-diphenylmethan
4,4'-Diamino-diphenylmethan
N,N,N,N'-Tetramethyl-4,4'-diamino-diphenylmethan
1,2-Di-[(2-methyl-phenyl)-amino]-ethan
1,2-Di-(phenylamino)-propan
(o-Tolyl)-biguanid
Di-[4-(1',3'-dimethyl-butyl)-phenyl)amin
tert-octyliertes N-Phenyl-1-naphthylamino
Gemisch aus mono- und dialkylierten tert-Butyl-/tert-Octyl-diphenylaminen.

Beispiele für Metallpassivatoren sind :

für Kupfer, z. B :

Benztriazol, Tetrahydrobenztriazol, 2-Mercaptobenzthiazol
2,5-Dimercaptothiadiazol, Salicyliden-propylendiamin, Salze von Salicylaminoguanidin.

Beispiele für Rost-Inhibitoren sind :

a) Organische Säuren, ihre Ester, Metallsalze und Anhydride, z. B. : N-Oleoyl-sarcosin, Sorbitanmono-oleat, Blei-naphthenat, Dodecenylbernsteinsäure-anhydrid, Alkenylbernsteinsäure-Halbester, 4-Nonylphenoxy-essigsäure.

b) Stickstoffhaltige Verbindungen, z. B. :
I. Primäre, sekundäre oder tertiäre aliphatische oder cycloaliphatische Amine und Amin-Salze von organischen und anorganischen Säuren, z. B. öllösliche Alkylammoniumcarboxylate.

II. Heterocyclische Verbindungen z. B. :
Substituierte Imidazoline und Oxazoline.

c) Phosphorhaltige Verbindungen, z. B. :
Aminsalze von Phosphorsäurepartialestern.

d) Schwefelhaltige Verbindungen, z. B. :
Barium-dinonylnaphthalin-sulfonate, Calciumpetroleum-sulfonate.

Beispiele für Viskositätsindex-Verbesserer sind z. B.

Polymethacrylate, Vinylpyrrolidon/Methacrylat-Copolymere, Polybutene, Olefin-Copolymere, Styrol/Acrylat-Copolymere.

Beispiele für Stockpunktniedriger sind z. B. :

Polymethacrylat, alkylierte Naphthalinderivate.

Beispiele für Dispergiermittel/Tenside sind z. B. :

Polybutenylbernsteinsäure-imide, Polybutenylphosphonsäurederivate, basische Magnesium-, Calcium-, und Bariumsulfonate und -phenolate.

Beispiele für Verschleissschutz-Additive sind z. B. :

Schwefel und/oder Phosphor und/oder Halogen enthaltende Verbindungen, wie geschwefelte pflanzliche Oele, Zinkdialkyldithiophosphate, Tritolyl-phosphat, chlorierte Paraffine, Alkyl- und Aryldisulfide.

Die erfindungsgemässen Verbindungen dienen als Zusätze für Schmiersysteme, insbesondere

Motorenöle. Sie weisen in Schmiersystemen Hochdruck-, Antiverschleiss-, Antioxidans- und Korrosionsinhibitor-Wirkungen auf. Ein besonderer Vorteil dieser Verbindungen ist, dass sie im Gegensatz zu Verbindungen mit vergleichbaren Eigenschaften Phosphor- und Zinn-frei sind ; wodurch dann auch die Nachverbrennung der Abgase nicht beeinträchtigt wird. Darüberhinaus enthalten diese Verbindungen keine hydrolytisch bzw. solvolytisch spaltbaren Bindungen und sind daher auch besonders stabil.

Um die Erfindung näher zu erläutern, seien die folgenden Herstellungsbeispiele gegeben, wobei die Beispiele 1 und 2 Zwischenprodukte zur Herstellung der Verbindungen der Formel I, dargestellt durch die Beispiele 3-27, sind :

Beispiele 1 : (vgl. Tabelle 1) tert-Octylglycidylthioether

Zu einer Mischung aus 219 Gewichtsteilen tert-Octylmercaptan und 135 Gewichtsteilen Epichlorhydrin wird bei 15 bis 20 °C unter Rühren und teilweiser Kühlung (besonders am Anfang der Zugabe) eine Lösung aus 66 Gewichtsteilen Natriumhydroxid, 300 Gewichtsteilen Wasser und 8 Gewichtsteilen Tetrabutylammoniumchlorid innerhalb von 70 Minuten zugetropft. Das Reaktionsgemisch wird 1 Stunde bei 50 °C nachgerührt, die wässerige Phase wird abgetrennt, und die organische Phase wird mit 200 Gewichtsteilen Wasser gewaschen. Schliesslich wird dann die organische Phase im Vakuum destilliert, und man erhält den tert-Octylglycidylthioether als farblose Flüssigkeit mit einem Siedepunkt von 74 bis 75 °C bei 0,02 Torr und einem Brechungsindex von $n_D^{20} = 1,4803$ ; die Ausbeute beträgt 250 Gewichtsteile, was 82 % der theoretischen Ausbeute entspricht.

Beispiel 3 : (vgl. Tabelle 1)

27,1 Gewichtsteile tert-Dodecylglycidylthioether werden zu einer Mischung aus 7,8 Gewichtsteilen 2-Mercaptoethanol und katalytischen Mengen Natriumhydrid bei 50-60 °C unter Rühren zugetropft (exotherme Reaktion). Nach beendeter Zugabe wird bei der gleichen Temperatur 1 Stunde nachgerührt und die folgende Verbindung als gelbe viskose Flüssigkeit mit einem Brechungsindex von $n_D^{20} = 1,5132$ in einer Ausbeute von 100 % erhalten

$$t\text{-}C_{12}H_{25}\text{-}S\text{-}CH_2\text{-}\underset{\displaystyle OH}{\underset{|}{CH}}\text{-}CH_2\text{-}S\text{-}CH_2\text{-}CH_2\text{-}OH$$

Beispiele 2, 4-27

Analog den in den Beispielen 1 und 3 beschriebenen Verfahren werden weitere Verbindungen hergestellt, die in der folgenden Tabelle zusammengestellt sind.

(Siehe Tabellen Seite 13 ff.)

Tabelle 1 :

| Bei-spiel Nr. | Formel | Bemerkungen | Physikalische Daten | |
|---|---|---|---|---|
| | | | Siedepunkt (°C) | $n_D^{20}$ |
| 1 | 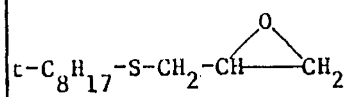 | farblose Flüssigkeit | 74-75 °C/ bei 2,66 Pa | 1,4803 |
| 2 | 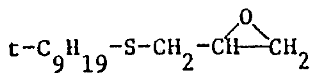 | farblose Flüssigkeit | 81-82°C/ bei 1,33 Pa | 1,4800 |
| 3 | 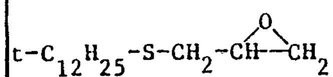 | farblose Flüssigkeit | 100-102°C/ bei 1,33 Pa | 1,4800 |
| 4 | 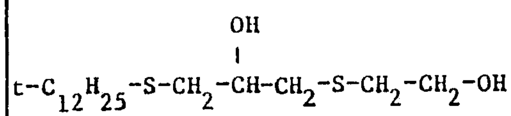 | viskose Flüssigkeit | | 1,5132 |
| 5 | 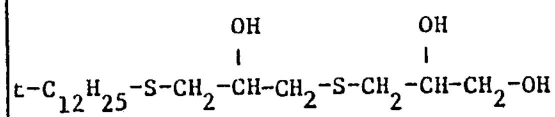 | viskose Flüssigkeit | | 1,5115 |
| 6 | 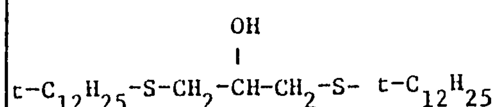 | viskose Flüssigkeit | | 1,4958 |

Tabelle 1 (Fortsetzung)

| Bei-spiel Nr. | Formel | Bemerkungen | Physikalische Daten | |
|---|---|---|---|---|
| | | | Siedepunkt (°C) | $n_D^{20}$ |
| 7 | $t-C_{12}H_{25}-S-CH_2-CH(OH)-CH_2-S-CH_2-CO-O-\ i-C_8H_{17}$ | viskose Flüssigkeit | | 1,4905 |
| 8 | $t-C_{12}H_{25}-S-CH_2-CH(OH)-CH_2-S-CH_2-$ 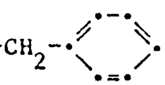 | viskose Flüssigkeit | | 1,5331 |
| 9 | $t-C_{12}H_{25}-S-CH_2-CH(OH)-CH_2-S-CH_2-CH_2-S-CH_2-CH(OH)-CH_2-S-\ t-C_{12}H_{25}$ | viskose Flüssigkeit | | 1,5210 |
| 10 | $t-C_{12}H_{25}-S-CH_2-CH(OH)-CH_2-S-S-CH_2-CH(OH)-CH_2-S-\ t-C_{12}H_{25}$ | | | |
| 11 | $t-C_{12}H_{25}-S-CH_2-CH(OH)-CH_2-S-P(=S)(O-i-C_3H_7)_2$ | viskose Flüssigkeit | | 1,5049 |

Tabelle 1 (Fortsetzung)

| Beispiel-Nr. | Formel | Bemerkungen | Physikalische Daten | |
|---|---|---|---|---|
| | | | Siedepunkt | $n_D^{20}$ |
| 12 | 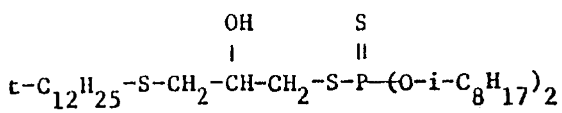 | | | 1,5056 |
| 13 | 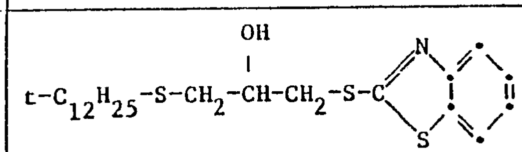 | viskose Flüssigkeit | | 1,5650 |
| 14 | 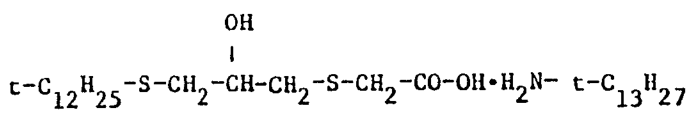 | viskose Flüssigkeit | | 1,4982 |
| 15 | 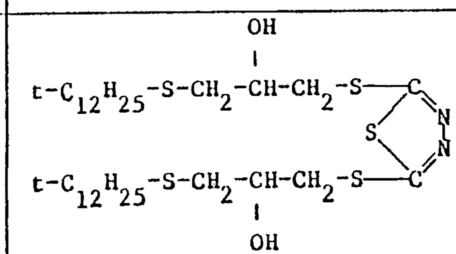 | viskose Flüssigkeit | | 1,5590 |

Tabelle 1 (Forsetzung)

| Bei-spiel Nr. | Formel | Bemerkungen | Physikalische Daten | |
|---|---|---|---|---|
| | | | Siedepunkt (°C) | $n_D^{20}$ |
| 16 | 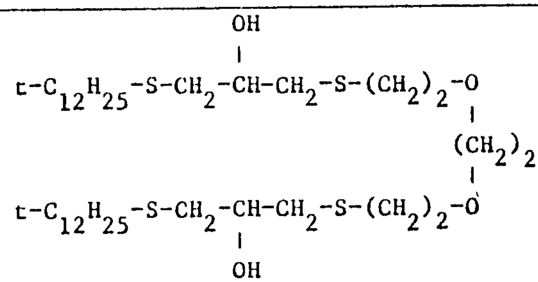 | farblose Flüssigkeit | | 1,5221 |
| 17 | 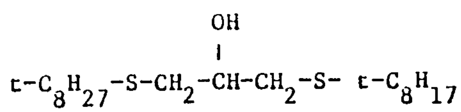 | farblose Flüssigkeit | 133-135°C/ bei 2,66 Pa | 1,5010 |
| 18 | 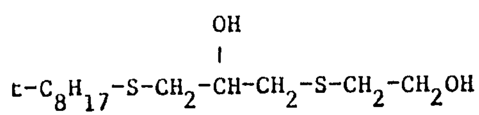 | viskose Flüssigkeit | | 1,5112 |

Tabelle 1 (Fortsetzung)

| Beispiel Nr. | Formel | Bemerkungen | Physikalische Daten | |
|---|---|---|---|---|
| | | | Siedepunkt (°C) | $n_D^{20}$ |
| 19 | 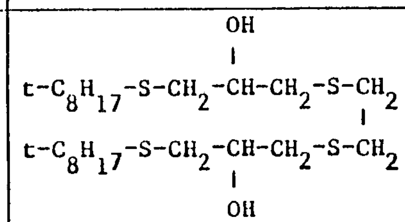 | viskose Flüssigkeit | | 1,5298 |
| 20 | 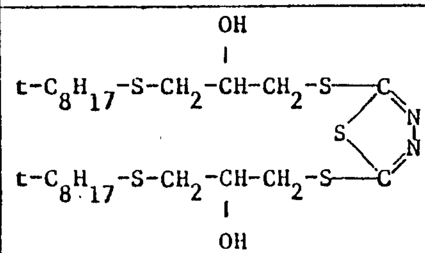 | farblose Flüssigkeit | | 1,5590 |
| 21 | 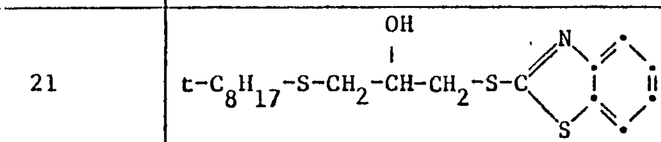 | viskose Flüssigkeit | | 1,5908 |
| 22 | 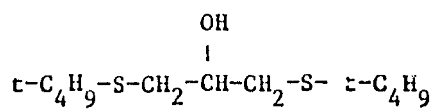 | farblose Flüssigkeit | 88-92°C/ bei 3,99 Pa | 1,4961 |

Tabelle 1 (Fortsetzung)

| Beispiel Nr. | Formel | Bemerkungen | Physikalische Daten | |
|---|---|---|---|---|
| | | | Siedepunkt (°C) | $n_D^{20}$ |
| 23 | $t-C_9H_{19}-S-CH_2-\overset{OH}{\overset{\vert}{CH}}-CH_2-S-\ t-C_9H_{19}$ | farblose Flüssigkeit | 143-147°C/ bei 6,65 Pa | 1,4990 |
| 24 | 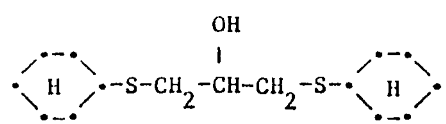 | | 168-169°C/ bei 2,66 Pa | 1,5392 |
| 25 | 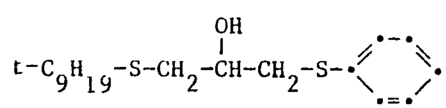 | | 160-161°C bei 5.32 Pa | 1.5477 |

Tabelle 1 (Fortsetzung)

| Beispiel Nr. | Formel | Bemerkungen | Physikalische Daten | |
|---|---|---|---|---|
| | | | Siedepunkt (°C) | $n_D^{20}$ |
| 26 | $t-C_9H_{19}S-CH_2-\overset{OH}{\overset{\vert}{C}H}-CH_2-S-$ 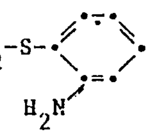 | | | 1,5638 |
| 27 | $t-C_9H_{19}-S-CH_2-\overset{OH}{\overset{\vert}{C}H}-CH_2-S-CH_2-\overset{OH}{\overset{\vert}{C}H}-$ 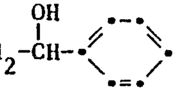 | | | 1,5446 |
| 28 | $(sec-C_4H_9-S-CH_2)_2\ CH-OH$ | | 97-98°C bei 5,32 Pa | 1,5000 |
| 29 | $t-C_9H_{19}-S-CH_2-\underset{\overset{\vert}{OH}}{CH}-CH_2-S-CH_2-CH_2-C_8F_{17}$ | | | 1,4179 |
| 30 | $t-C_9H_{19}-S-CH_2-\underset{\overset{\vert}{OH}}{CH}-CH_2-S-$ 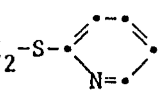 | | | 1,5467 |

Tabelle 1 (Fortsetzung)

| Beispiel Nr. | Formel | Bemerkungen | Physikalische Daten: Siedepunkt (°C) | Physikalische Daten: $n_D^{20}$ |
|---|---|---|---|---|
| 31 | 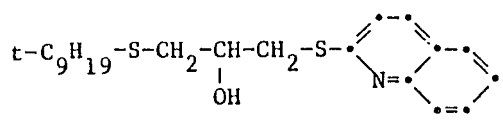 | | | 1,5845 |
| 32 | 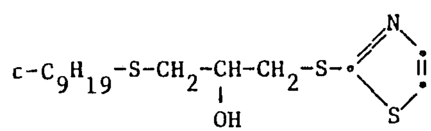 | | | 1,5491 |
| 33 | $n\text{-}C_{12}H_{25}\text{-}S\text{-}CH_2\text{-}\underset{OH}{CH}\text{-}CH_2\text{-}S\text{-}CH_2\text{-}CH_2\text{-}OH$ | fester Stoff | Schmelzpunkt 46-48° | |
| 34 | $t\text{-}C_9H_{19}\text{-}S\text{-}CH_2\text{-}\overset{OH}{CH}\text{-}CH_2\text{-}S\text{-}CH_2\text{-}CH_2\text{-}OH$ | | | 1,5160 |
| 35 | $t\text{-}C_9H_{19}\text{-}S\text{-}CH_2\text{-}\overset{OH}{CH}\text{-}CH_2\text{-}S\text{-}\overset{S}{\overset{\parallel}{P}}(O\text{-}i\text{-}C_3H_7)_2$ | | | 1,5122 |
| 36 | 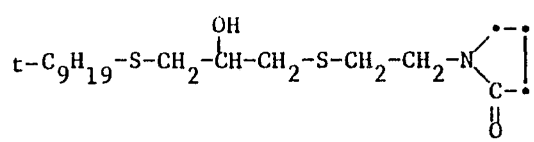 | | | 1,5224 |

Tabelle 1 (Fortsetzung)

| Beispiel Nr. | Formel | Bemerkungen | Physikalische Daten Siedepunkt (°C) | Physikalische Daten $n_D^{20}$ |
|---|---|---|---|---|
| 37 | $t-C_{12}H_{25}-S-CH_2-CH(OH)-CH_2-S-CH_2-CH_2-$ 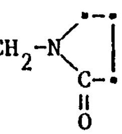 | | | 1,5143 |
| 38 | $t-C_9H_{19}-S-CH_2-CH(OH)-CH_2-S-CH_2-CH_2-O-n-C_4H_9$ | | 153-156°C bei 6.65 Pa | 1,4948 |
| 39 | $t-C_{12}H_{25}-S-CH_2-CH(OH)-CH_2-S-CH_2-CO-N(CH_2-CH_2-OH)_2$ | | | 1,5170 |
| 40 | $n-C_8H_{17}-S-CH_2-CH(OH)-CH_2-S-$ 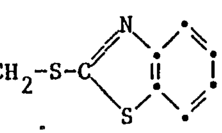 | | | 1.5830 |
| 41 | $t-C_9H_{19}-S-CH_2-CH(OH)-CH_2-S-CH_2-CO-NH-$ 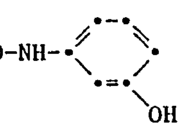 | | | 1,561 |

Das folgende Anwendungsbeispiel dient der näheren Erläuterung der anwendungstechnisch erhaltenen Ergebnisse.

Anwendungsbeispiel 1

Mit dem Shell-Vierkugel-Apparat (IP 239/73 Extreme pressure and wear lubricant test for oils and greases-four ballmachine) werden folgende Werte bestimmt :

1. W.L. = Weld load (Schweisslast). Das ist die Last, bei der die 4 Kugeln innerhalb von 10 Sekunden zusammenschweissen.
2. W.S.D. = Wear Scar Diameter in mm : Das ist der mittlere Verschleissdurchmesser bei einer Belastung von 400 N während 10 Minuten.

Als Testflüssigkeit für die Wirksamkeit der Additive wird ein Basisöl der Viskosität ISO-VH 100 mit niedrigen Aromatengehalt und 0,035 % S verwendet.

Tabelle 2

| Additiv: Beispiel Nr. (vgl. Tabelle 1) | Test mit dem Shell-Vierkugel-Apparat | | | |
|---|---|---|---|---|
| | W.L. (N) | | W.S.D. 10 min. (mm) | |
| | 1 % Additiv | 2,5 % Additiv | 0,25 % Additiv | 1,0 % Additiv |
| 4 | 2200 | 2400 | 0,60 | 0,50 |
| 5 | 1800 | | | 0,75 |
| 6 | 2200 | 2400 | 0,60 | 0,50 |
| 7 | 1800 | | | 0,75 |
| 8 | 2200 | 2400 | 0,50 | 0,50 |
| 9 | 2200 | 2200 | 0,50 | 0,70 |
| 10 | 2200 | 2600 | 0,70 | 0,75 |
| 11 | 2200 | 2400 | 0,50 | 0,50 |
| 12 | 2200 | 2400 | 0,50 | 0,50 |
| 13 | 2000 | 2200 | 0,50 | 0,50 |
| 16 | 2000 | 2200 | 0,55 | 0,60 |
| 17 | 2000 | 2400 | 0,50 | 0,55 |
| 19 | 2200 | 2400 | 0,60 | 0,65 |
| 20 | 2400 | 2800 | 0,55 | 0,65 |
| 21 | 2350 | 2700 | | |
| 22 | 2000 | 2200 | 0,50 | 0,55 |
| 23 | 2000 | 2050 | | |

**Patentansprüche**

1. Zusammensetzungen, enthaltend ein Schmiermittel oder eine Hydraulikflüssigkeit und wenigstens eine Verbindung der Formel I

$$R-S-CH_2-\underset{\displaystyle OH}{\underset{|}{CH}}-CH_2-S-R^4 \qquad (I)$$

worin R ein Radikal der Form

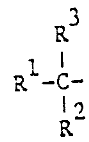

sein kann, wobei $R^1$, $R^2$ und $R^3$ unabhängig voneinander $C_1$-$C_{18}$-Alkyl sind und zusammen nicht mehr als 22 C-Atome besitzen und $R^2$ und $R^3$ ausserdem Wasserstoff sind, oder worin R $C_5$-$C_6$-Cycloalkyl, unsubstituiertes oder durch $C_1$-$C_4$-Alkyl substituiertes Phenyl oder Naphthyl, Benzyl, Furyl, Thienyl, Morpholinyl, Imidazolyl, Thiazolyl, Oxazolyl, Imidazolinyl, Thiazolinyl, Oxazolinyl, Benzimidazolinyl, Benzthiazolinyl, Benzoxazolinyl ist, und worin $R^4$ unsubstituiertes oder durch $-NH_2$ substituiertes Phenyl, unsubstituiertes oder durch Phenyl, $-NH_2$, 2-Oxopyrrolidino, Cyano, Perfluoro-$C_1$-$C_8$-Alkyl oder eine oder zwei OH-Gruppen substituiertes $C_1$-$C_{16}$-Alkyl, das gegebenenfalls durch $-O-$ oder $-S-$ unterbrochen sein kann, oder $C_5$-$C_6$-Cycloalkyl ist oder $R^4$ $-(CH_2)_m-S-CH_2-CH(OH)-CH_2-S-(C_1$-$C_{16}$-Alkyl) mit m gleich 0 bis 6 ist, oder $R^4$ $-(CH_2)_n-C(O)-O-R^5$ ist, wobei n gleich 1 oder 2 und $R^5$ Wasserstoff, $C_1$-$C_{16}$-Alkyl oder Alkalimetall ist, oder worin $R^4$ $-CH[-CO-OR^5]$ $[-CH_2-CO-OR^5]$ bedeutet, wobei $R^5$ die oben gegebene Bedeutung aufweist, oder worin $R^4$ $-(CH_2)_r-C(O)-OH \cdot H_2N$-($C_8$-$C_{16}$-Alkyl) oder $-(CH_2)_r-C(O)-OH \cdot N-(CH_2-CH_2-OH)_3$ mit r gleich 1 oder 2 oder $-P(X)-[O-R^6]_2$ darstellt, wobei X = 0 oder S sein kann, und $R^6$ $C_1$-$C_{16}$-Alkyl, Phenyl oder Tolyl ist, oder worin $R^4$ α- oder β-Naphthyl, Benzthiazolyl, Benzimidazolyl, Benzoxazolyl, Thiazolyl, Thiazolinyl, Triazolyl, Tetrazolyl, Pyridyl, Chinolyl, Imidazolyl, Imidazolinyl, Oxazolinyl, $-SO_2-O-$(Alkalimetall), $-C_6H_4-C(O)-O-$(Alkalimetall), 2-Oxo-4-hydroxy-3-penten-3-yl oder $-(CH_2)_s-R^7$ ist, wobei s gleich 1 bis 4 ist und $R^7$ Benzoxazolyl, Benzimidazolyl, Benzthiazolyl, Thiazolinyl, Imidazolinyl oder Oxazolinyl darstellt, oder worin $R^4$ $-(CH_2)_t-CO-N(R^8)$ $(R^9)$ bedeutet, worin t gleich 1 oder 2 ist und $R^8$ $C_1$-$C_{16}$-Alkyl, das gegebenenfalls durch $-OH$ substituiert sein kann, Phenyl, 3-Hydroxyphenyl oder α-Naphthyl und $R^9$ Wasserstoff oder $R^8$ ist, oder worin $R^4$ $-CH_2-CH(OH)-CH_2-S-R^{10}$ ist, wobei $R^{10}$ Wasserstoff oder $C_1$-$C_{16}$-Alkyl ist, oder worin $R^4$ einen Rest $-R^{11}-S-CH_2-CH(OH)-CH_2-S-R$ darstellt, wobei R die oben gegebene Bedeutung hat und $R^{11}$ ein Radikal $-(CH_2)_2-O-(CH_2)_2O-(CH_2)_2-$, o- oder m-Phenylen, Thiadiazol-2,5-ylen oder $-(CH_2)_u-$ mit u gleich 0 bis 8 ist oder ein Radikal der Formeln

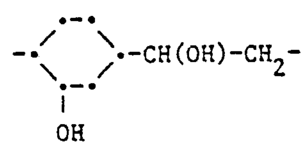

oder

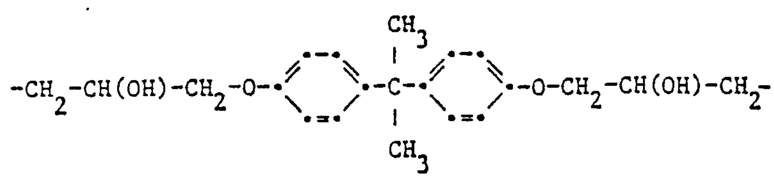

darstellt.

2. Zusammensetzungen gemäss Anspruch 1, enthaltend ein Schmiermittel oder eine Hydraulikflüssigkeit und wenigstens eine Verbindung der Formel II

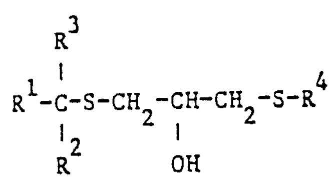

(II)

worin $R^1$, $R^2$ und $R^3$ zusammen mit dem C-Atom, an das sie gebunden sind, $C_4$-$C_{20}$-Alkyl sind, wobei keiner dieser Substituenten $R^1$, $R^2$ und $R^3$ Wasserstoff sein darf, und worin $R^4$ unsubstituiertes oder durch $-NH_2$ substituiertes Phenyl, unsubstituiertes oder durch Phenyl, $-NH_2$, 2-Oxopyrrolidino, Cyano, Perfluoro-$C_1$-$C_8$-Alkyl, oder eine oder zwei OH-Gruppen substituiertes $C_1$-$C_{16}$-Alkyl, das gegebenenfalls durch $-O-$ oder $-S-$ unterbrochen sein kann, oder $C_5$-$C_6$-Cycloalkyl ist oder $R^4$ $-(CH_2)_m-S-CH_2-CH(OH)-CH_2-S-(C_1$-$C_{16}$-Alkyl) mit m gleich 0 bis 6, oder $-(CH_2)_n-C(O)-O-R^5$ ist, wobei n gleich 1 oder 2 und $R^5$ Wasserstoff, $C_1$-$C_{16}$-Alkyl oder Alkalimetall ist, oder worin $R^4$ $-CH[-CO-OR^5]$ $[-CH_2-CO-OR^5]$ bedeutet, wobei $R^5$ die oben gegebene Bedeutung aufweist, oder worin $R^4$ $-(CH_2)_r-C(O)-OH \cdot H_2N-(C_8$-$C_{16}$-Alkyl) oder $-(CH_2)_r-C(O)-OH \cdot N-(CH_2-CH_2-OH)_3$ mit r gleich 1 oder 2, oder $-P(X)-[O-R^6]_2$ darstellt, wobei X = 0 oder = S sein kann und $R^6$ $C_1$-$C_{16}$-Alkyl, Phenyl oder Tolyl ist, oder worin $R^4$ α- oder β-Naphthyl,

Benzthiazolyl, Benzimidazolyl, Benzoxazolyl, Thiazolyl, Thiazolinyl, Pyridyl, Chinolyl, Imidazolinyl, Oxazolinyl, —$SO_2$—O—(Alkalimetall), —$C_6H_4$—C(O)—O—(Alkalimetall), 2-Oxo-4-hydroxy-3-penten-3-yl, —$(CH_2)_s$—$R^7$ ist, wobei s gleich 1 bis 4 ist und $R^7$ Benzoxazolyl, Benzimidazolyl, Benzthiazolyl, Thiazolinyl, Imidazolinyl oder Oxazolinyl darstellt, oder worin $R^4$ —$(CH_2)_t$—CO—N($R^8$) ($R^9$) bedeutet, wobei t gleich 1 oder 2 ist und $R^8$ $C_1$-$C_{16}$-Alkyl, das gegebenenfalls durch —OH substituiert sein kann, Phenyl, 3-Hydroxyphenyl oder α-Naphthyl und $R^9$ Wasserstoff oder $R^8$ ist, oder worin $R^4$ —$CH_2$—CH(OH)—$CH_2$—S—$R^{10}$ ist, wobei $R^{10}$ Wasserstoff oder $C_1$-$C_{16}$-Alkyl ist, oder worin $R^4$ einen

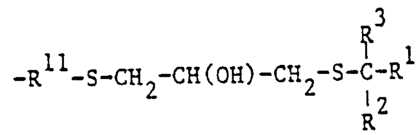

darstellt, wobei $R^1$, $R^2$ und $R^3$ die oben gegebene Bedeutung haben und $R^{11}$ ein Radikal —$(CH_2)_2$—O—$(CH_2)_2$—O—$(CH_2)_2$—, o- oder m-Phenylen, Thiadiazol-2,5-ylen der —$(CH_2)_u$— mit u gleich 0 bis 8, bevorzugt 2, ist oder ein Radikal der Formeln

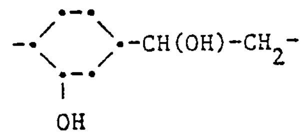 oder

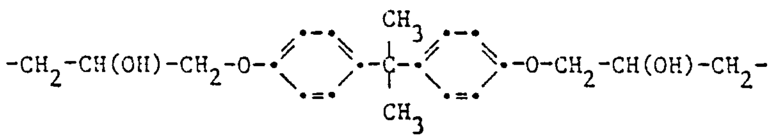

darstellt.

3. Zusammensetzungen gemäss Anspruch 2, enthaltend ein Schmiermittel oder eine Hydraulikflüssigkeit und wenigstens eine Verbindung der Formel II, worin $R^1$, $R^2$ und $R^3$ zusammen mit dem C-Atom, an das sie gebunden sind, $C_4$-$C_{20}$-Alkyl sind, wobei keiner dieser Substituenten $R^1$, $R^2$ und $R^3$ Wasserstoff sein darf, und worin $R^4$ unsubstituiertes oder durch —$NH_2$ substituiertes Phenyl, unsubstituiertes oder durch Phenyl, —$NH_2$, 2-Oxopyrrolidino, Cyano oder eine oder zwei OH-Gruppen substituiertes $C_1$-$C_{13}$-Alkyl, das gegebenenfalls durch —O— oder —S— unterbrochen sein kann, ist oder worin $R^4$ —$(CH_2)_m$—S—$CH_2$—CH(OH)—$CH_2$—S—($C_1$-$C_{16}$)-Alkyl) mit m gleich 0 bis 4, oder —$(CH_2)_n$—CO—O—$R^5$ ist, wobei $R^5$ Wasserstoff, Kalium oder $C_4$-$C_{12}$-Alkyl und n gleich 1 oder 2 ist, oder worin $R^4$ —$(CH_2)_r$—CO—OH·$H_2N$—($C_{10}$-$C_{16}$-Alkyl) mit r gleich 1 oder 2, —P(S)—[O—$R^6$]$_2$ darstellt, und $R^6$ $C_1$-$C_8$-Alkyl oder Phenyl ist, oder worin $R^4$ α-Naphthyl, Thiazolyl, Benzthiazolyl, Benzimidazolyl, Benzoxazolyl, Pyridyl, Chinolyl oder —$(CH_2)_s$—$R^7$ ist, wobei s gleich 1 oder 2 ist und $R^7$ Benzoxazolyl, Benzimidazolyl, Benzthiazolyl, Thiazolinyl, Imidazolinyl oder Oxazolinyl ist, oder worin $R^4$ —$(CH_2)_t$—CO—N($R^8$) ($R^9$) ist, wobei t gleich 1 oder 2 ist und $R^8$ $C_1$-$C_4$-Alkyl, das gegebenenfalls durch —OH substituiert sein kann, Phenyl oder α-Naphthyl ist, und $R^9$ Wasserstoff oder $R^8$ ist, oder worin $R^4$ —$CH_2$—CH(OH)—$CH_2$—S—$R^{10}$ ist, wobei $R^{10}$ $C_1$-$C_{14}$-Alkyl ist, oder worin $R^4$ einen Rest

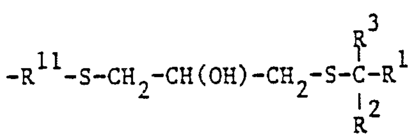

darstellt, wobei $R^1$, $R^2$ und $R^3$ die oben gegebene Bedeutung haben und $R^{11}$ —$(CH_2)_2$—O—$(CH_2)_2$—O—$(CH_2)_2$—, o- oder m-Phenylen, Thiadiazol-2,5-ylen oder —$(CH_2)_u$— mit u gleich 0 bis 4, bevorzugt 2, ist.

4. Zusammensetzungen gemäss Anspruch 3, enthalten ein Schmiermittel oder eine Hydraulikflüssigkeit und wenigstens eine Verbindung der Formel II, worin $R^1$, $R^2$ und $R^3$ zusammen mit dem C-Atom, an das sie gebunden sind, C-$C_{14}$-Alkyl sind, wobei keiner dieser Substituenten $R^1$, $R^2$ und $R^3$ Wasserstoff sein darf, und worin $R^4$ phenyl, —$CH_2$—$CH_2$—$NH_2$, —$CH_2$—$CH_2$—OH, —$CH_2$—CH(OH)—$CH_2$—OH, tertiäres $C_4$-$C_{14}$-Alkyl, —$(CH_2)_2$—S—CH(OH)—$CH_2$—S—(tert.-$C_8$-$C_{12}$-Alkyl), —$CH_2$—COOH —$CH_2$—CO—O—(i-$C_8H_{17}$), —$CH_2$—CO—OH·$H_2N$—(tert.-$C_{10}$-$C_{16}$-Alkyl), —P(S)—[O-(i-$C_3H_7$)]$_2$, —P(S)—[O-(i-$C_8H_{17}$)]$_2$, α-Naphthyl, Benzthiazolyl, Benzimidazolyl, Thiazolyl oder —$CH_2$—CH(OH)—$CH_2$—S—$R^{10}$ ist, wobei $R^{10}$ tertiäres $C_4$-$C_{14}$-Alkyl ist, oder worin $R^4$ einen Rest

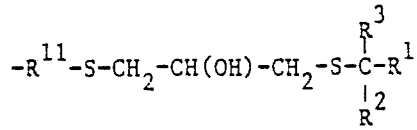

darstellt, wobei $R^1$, $R^2$ und $R^3$ die oben gegebene Bedeutung haben und $R^{11}$ $-(CH_2)_2-O-(CH_2)_2-O-(CH_2)_2-$, o-Phenylen, Thiadiazol-2,5-ylen oder $-(CH_2)_u-$ mit u gleich 0 bis 2 ist.

5. Verbindungen der Formel II nach Anspruch 2, worin $R^1$, $R^2$ und $R^3$ zusammen mit dem C-Atom, an das sie gebunden sind, $C_4$-$C_{20}$-Alkyl sind, wobei keiner dieser Substituenten $R^1$, $R^2$ und $R^3$ Wasserstoff sein darf, und worin $R^4$ unsubstituiertes oder durch $-NH_2$ substituiertes Phenyl, unsubstituiertes $C_1$-$C_9$-Alkyl oder durch Phenyl, $-NH_2$, 2-Oxopyrrolidino, Cyano, Perfluoro-$C_1$-$C_8$-Alkyl oder eine oder zwei OH-Gruppen substituiertes $C_1$-$C_{16}$-Alkyl ist, das gegebenenfalls durch $-O-$ oder $-S-$ unterbrochen sein kann, oder worin $R^4$ $-(CH_2)_m-S-CH_2-CH(OH)-CH_2-S-(C_1$-$C_{16}$-Alkyl) mit m gleich 0 bis 6, oder $-(CH_2)_n-C(O)-O-R^5$ ist, wobei n gleich 1 oder 2 und $R^5$ Wasserstoff, $C_1$-$C_{16}$-Alkyl oder Alkalimetall ist, oder worin $R^4$ $-CH[-CO-OR^5]$ $[-CH_2-CO-OR^5]$ bedeutet, wobei $R^5$ die oben gegebene Bedeutung aufweist, oder worin $R^4$ $-(CH_2)_r-C(O)-OH\cdot H_2N$-($C_8$-$C_{16}$-Alkyl) oder $-(CH_2)_r-C(O)-OH\cdot N(-CH_2-CH_2-OH)_3$ mit r gleich 1 oder 2, oder $-P(X)-[O-R^6]_2$ darstellt, wobei X = O oder = S sein kann, und $R^6$ $C_1$-$C_{16}$-Alkyl, Phenyl oder Tolyl ist, oder worin $R^4$ α- oder β-Naphthyl, Benzthiazolyl, Benzimidazolyl, Benzoxazolyl, Thiazolyl, Thiazolinyl, Pyridyl, Chinolyl, Imidazolyl, Oxazolinyl, $-SO_2-O-$(Alkalimetall), $-C_6H_4-C(O)-O-$(Alkalimetall), 2-Oxo-4-hydroxy-3-penten-3-yl, oder $-(CH_2)_s-R$ ist, wobei s gleich 1 bis 4 ist und $R^7$ Benzoxazolyl, Benzimidazolyl, Benzthiazolyl, Thiazolinyl, Imidazolinyl oder Oxazolinyl darstellt, oder worin $R^4$ $-(CH_2)_t-CO-N(R^8)$ $(R^9)$ bedeutet wobei t gleich 1 oder 2 ist und $R^8$ $C_1$-$C_{16}$-Alkyl, das gegebenenfalls durch $-OH$ substituiert sein kann, Phenyl, 3-Hydroxyphenyl oder α-Naphthyl ist und $R^9$ Wasserstoff oder $R^8$ ist, oder worin $R^4$ $-CH_2-CH(OH)-CH_2-S-R^{10}$ ist, wobei $R^{10}$ Wasserstoff oder $C_1$-$C_{16}$-Alkyl ist, oder worin $R^4$ einen Rest

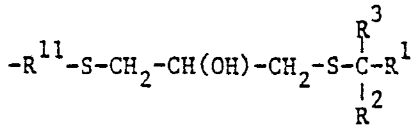

darstellt, wobei $R^1$, $R^2$ und $R^3$ die oben gegebene Bedeutung haben und $R^{11}$ ein Radikal $-(CH_2)_2-O-(CH_2)_2-O-(CH_2)_2-$, o- oder m-Phenylen, Thiadiazol-2,5-ylen oder $-(CH_2)_u-$ mit u gleich 0 bis 8 ist oder ein Radikal der Formeln

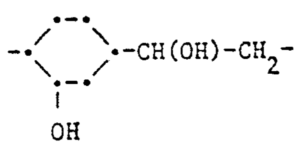 oder

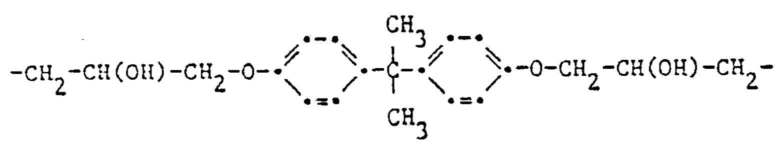

darstellt.

6. Verbindungen gemäss Anspruch 5, worin in Formel II $R^1$, $R^2$ und $R^3$ zusammen mit dem C-Atom, an das sie gebunden sind, $C_4$-$C_{20}$-Alkyl sind, wobei keiner dieser Substituenten $R^1$, $R^2$ und $R^3$ Wasserstoff sein darf, und worin $R^4$ unsubstituiertes oder durch $-NH_2$ substituiertes Phenyl, unsubstituiertes $C_4$-$C_9$-Alkyl oder durch Phenyl, $-NH_2$, 2-Oxopyrrolidino, Cyano oder eine oder zwei OH-Gruppen substituiertes $C_1$-$C_{13}$-Alkyl, $-(CH_2)_m-S-CH_2-CH(OH)-CH_2-S-(C_1$-$C_{16}$-Alkyl) mit m gleich 0 bis 4, oder $-(CH_2)_n-CO-O-R^5$ ist, wobei $R^5$ Wasserstoff, Kalium oder $C_4$-$C_{12}$-Alkyl und n gleich 1 oder 2 ist, oder worin $R^4$ $-(CH_2)_r-CO-OH\cdot H_2N(C_{10}$-$C_{16}$-Alkyl) mit r gleich 1 oder 2, $-P(S)-[O-R^6]_2$, wobei $R^6$ $C_1$-$C_8$-Alkyl oder Phenyl ist, oder worin $R^4$ α-Naphthyl, Thiazolyl, Benzthiazolyl, Benzimidazolyl, Benzoxazolyl, oder $-(CH_2)_s-R^7$ ist, wobei s gleich 1 oder 2 ist und $R^7$ Benzoxazolyl, Benzimidazolyl, Benzthiazolyl, Thiazolinyl, Imidazolinyl oder Oxazolinyl ist, oder worin $R^4$ $-(CH_2)_t-CO-N-(R^8)$ $(R^9)$ ist, wobei t gleich 1 oder 2 ist und $R^8$ $C_1$-$C_4$-Alkyl, das gegebenenfalls durch $-OH$ substituiert sein kann, Phenyl oder α-Naphthyl ist und $R^9$ Wasserstoff oder $R^8$ ist, oder worin $R^4$ $-CH_2-CH(OH)-CH_2-S-R^{10}$ ist, wobei $R^{10}$ $C_1$-$C_{14}$-Alkyl ist, oder worin $R^4$ einen Rest

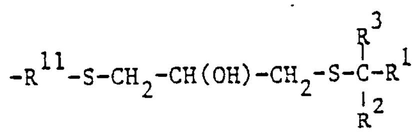

darstellt, wobei $R^1$, $R^2$ und $R^3$ die oben gegebene Bedeutung haben und $R^{11}$ $-(CH_2)_2-O-(CH_2)_2-O-(CH_2)_2-$, o- oder m-Phenylen, Thiadiazol-2,5-ylen oder $-(CH_2)_u-$ mit u gleich 0 bis 4, bevorzugt 2, ist.

7. Verbindungen gemäss Anspruch 6, worin in Formel II $R^1$, $R^2$ und $R^3$ zusammen mit dem C-Atom, an das sie gebunden sind, $C_4$-$C_{14}$-Alkyl sind, wobei keiner dieser Substituenten $R^1$, $R^2$ und $R^3$ Wasserstoff sein darf, und worin $R^4$ Phenyl, $-CH_2-CH_2-NH_2$, $-CH_2-CH_2-OH$, $-CH_2-CH(OH)-CH_2-OH$, tertiäres $C_4$-$C_9$-Alkyl, $-(CH_2)_2-S-CH(OH)-CH_2-S-$(tert.-$C_8$-$C_{12}$-Alkyl), $-CH_2COOH$, $-CH_2-CO-O-$(i-$C_8H_{17}$), $-CH_2-CO-OH\cdot H_2N-$(tert.-$C_{10}$-$C_{16}$-Alkyl), $-P(S)-[O-$(i-$C_3H_7$)$]_2$, $-P(S)-[O-$(i-$C_8H_{17}$)$]_2$, α-Naphthyl, Benzthiazolyl, Benzimidazolyl, Thiazolyl oder $-CH_2-CH(OH)-CH_2-S-R^{10}$ ist, wobei $R^{10}$ tertiäres $C_4$-$C_{14}$-Alkyl ist, oder worin $R^4$ einen Rest

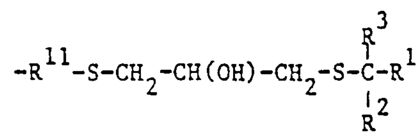

darstellt, wobei $R^1$, $R^2$ und $R^3$ die oben gegebene Bedeutung haben und $R^{11}$ $-(CH_2)_2-O-(CH_2)_2-O-(CH_2)_2-$, o-Phenylen, Thiadiazol-2,5-ylen oder $-(CH_2)_u-$ mit u gleich 0 bis 2 ist.

8. Verwendung von Verbindungen der Formel I gemäss Anspruch 1 als Additive in Schmierstoffen.

## Claims

1. A composition containing a lubricant or a hydraulic fluid and at least one compound of the formula I

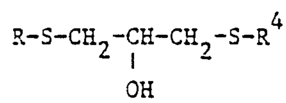

(I)

in which R can be a radical of the form

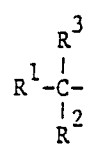

where $R^1$, $R^2$ and $R^3$ independently of one another are each $C_1$-$C_{18}$ alkyl and together contain no more than 22 C atoms, and $R^2$ and $R^3$ are in addition hydrogen, or in which R is $C_5$-$C_6$ cycloalkyl, phenyl or naphthyl unsubstituted or substituted by $C_1$-$C_4$ alkyl, or is benzyl, furyl, thienyl, morpholinyl, imidazolyl, thiazolyl, oxazolyl, imidazolinyl, thiazolinyl, oxazolinyl, benzimidazolinyl, benzthiazolinyl or benzoxazolinyl, and in which $R^4$ is phenyl unsubstituted or substituted by $-NH_2$, or is $C_1$-$C_{16}$-alkyl which is unsubstituted or substituted by phenyl, $-NH_2$, 2-oxopyrrolidino, cyano, perfluoro-$C_1$-$C_8$ alkyl or one or two OH groups, and which can be interrupted by $-O-$ or $-S-$, or is $C_5$-$C_6$ cycloalkyl, or $R^4$ is $-(CH_2)_m-S-CH_2-CH(OH)-CH_2-S-$($C_1$-$C_{16}$ alkyl), m being zero to 6, or $R^4$ is $-(CH_2)_n-C(O)-O-R^5$ where n is 1 or 2, and $R^5$ is hydrogen, $C_1$-$C_{16}$ alkyl or an alkali metal, or in which $R^4$ is $-CH[-CO-OR^5]$ $[-CH_2-CO-OR^5]$ where $R^5$ has the meaning given above, or in which $R^4$ is $-(CH_2)_r-C(O)-OH\cdot H_2N-(C_8$-$C_{16}$ alkyl) or $-(CH_2)_r-C(O)-OH\cdot N-(CH_2-CH_2-OH)_3$, r being 1 or 2, or is $-P(X)-[O-R^6]_2$, where X can be O or S, and $R^6$ is $C_1$-$C_{16}$ alkyl, phenyl or tolyl, or in which $R^4$ is α- or β-naphthyl, benzothiazolyl, benzimidazolyl, benzoxazolyl, thiazolyl, thiazolinyl, triazolyl, tetrazolyl, pyridyl, quinolyl, imidazolyl, imidazolinyl, oxazolinyl, $-SO_2-O-$(alkali metal), $-C_6H_4-C(O)-O-$(alkali metal), 2-oxo-4-hydroxy-3-penten-3-yl or $-(CH_2)_s-R^7$, where s is 1 to 4, and $R^7$ is benzoxazolyl, benzimidazolyl, benzothiazolyl, thiazolinyl, imidazolinyl or oxazolinyl, or in which $R^4$ is $-(CH_2)_t-CO-N-(R^8)$ $(R^9)$, in which t is 1 or 2, and $R^8$ is $C_1$-$C_{16}$ alkyl which can be substituted by $-OH$,

or is phenyl, 3-hydroxyphenyl or α-naphthyl, and $R^9$ is hydrogen or $R^8$, or in which $R^4$ is $—CH_2—CH(OH)—CH_2—S—R^{10}$, where $R^{10}$ is hydrogen or $C_1$-$C_{16}$-alkyl, or in which $R^4$ is a radical $—R^{11}—S—CH_2—CH(OH)—CH_2—S—R$ where R has the meaning given above, and $R^{11}$ is a radical $—(CH_2)_2—O—(CH_2)_2—O—(CH_2)_2—$, o- or m-phenylene, thiadiazol-2,5-ylene or $—(CH_2)_u—$, u being zero to 8, or a radical of the formulas

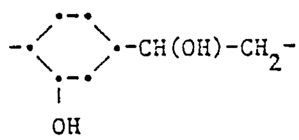 or

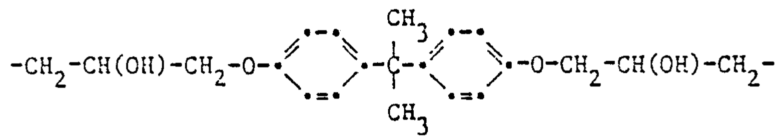

2. A composition according to claim 1, containing a lubricant or a hydraulic fluid and at least one compound of the formula II

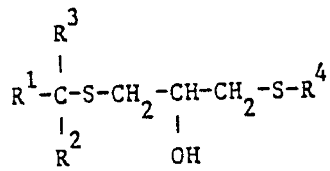 (II)

in which $R^1$, $R^2$ and $R^3$ together with the C atom to which they are bound are $C_4$-$C_{20}$ alkyl, and none of these substituents $R^1$, $R^2$ and $R^3$ may be hydrogen, and in which $R^4$ is phenyl unsubstituted or substituted by $—NH_2$, or is $C_1$-$C_{16}$alkyl which is unsubstituted or substituted by phenyl, $—NH_2$ 2-oxopyrrolidino, cyano, perfluoro-$C_1$-$C_8$-alkyl or one or two OH groups, and which can be interrupted by —O— or —S—, or is $C_5$-$C_6$-cycloalkyl, or $R^4$ is $—(CH_2)_m—S—CH_2—CH(OH)—CH_2—S—(C_1$-$C_{16}$-alkyl), m being zero to 6, or $—(CH_2)_n—C(O)—O—R^5$, where n is 1 or 2, and $R^5$ is hydrogen, $C_1$-$C_{16}$ alkyl or an alkali metal, or in which $R^4$ is $—CH[—CO—OR^5]$ $[—CH_2—CO—OR^5]$, where $R^5$ has the meaning defined above, or in which $R^4$ is $—(CH_2)_r—C(O)—OH·H_2N—(C_8$-$C_{16}$ alkyl) or $—(CH_2)_r—C(O)—OH·N—(CH_2—CH_2—OH)_3$, r being 1 or 2, or is $—P(X)—[O—R^6]_2$ where X can be O or = S, and $R^6$ is $C_1$-$C_{16}$ alkyl, phenyl or tolyl, or in which $R^4$ is α- or β-naphthyl, benzothiazolyl, benzimidazolyl, benzoxazolyl, thiazolyl, thiazolinyl, pyridyl, quinolyl, imidazolinyl, oxazolinyl, $—SO_2—O—$(alkali metal), $C_6H_4—C(O)—O$-(alkali metal), 2-oxo-4-hydroxy-3-penten-3-yl), $—(CH_2)_s—R^7$, where s is 1 to 4, and $R^7$ is benzoxazolyl, benzimidazolyl, benzothiazolyl, thiazolinyl, imidazolinyl or oxazolinyl, or in which $R^4$ is $—(CH_2)_t—CO—N(R^8)$ $(R^9)$, where t is 1 or 2, and $R^8$ is $C_1$-$C_{16}$ alkyl which can be substituted by —OH, or is phenyl, 3-hydroxyphenyl or α-naphthyl, and $R^9$ is hydrogen or $R^8$, or in which $R^4$ is $—CH_2—CH(OH)—CH_2—S—R^{10}$, where $R^{10}$ is hydrogen or $C_1$-$C_{16}$ alkyl, or in which $R^4$ is a radical

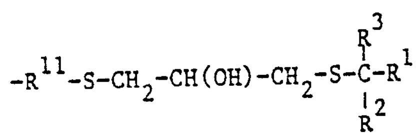

where $R^1$, $R^2$ and $R^3$ have the meanings defined above, and $R^{11}$ is a radical $—(CH_2)_2—O—(CH_2)_2—O—(CH_2)_2—$, o- or m-phenylene, thiadiazol-2,5-ylene or $—(CH_2)_u$, u being zero to 8, preferably 2, or a radical of the formulae

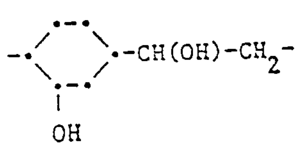 or

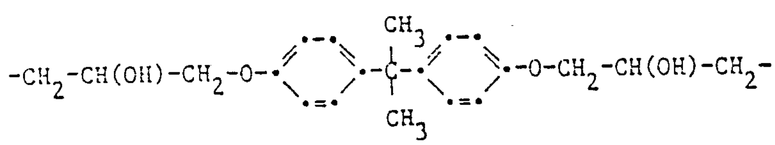

3. A composition according to claim 2, containing a lubricant or a hydraulic fluid and at least one compound of the formula II in which $R^1$, $R^2$ and $R^3$ together with the C atom to which they are bound are $C_4$-$C_{20}$ alkyl, and none of these substituents $R^1$, $R^2$ and $R^3$ may be hydrogen, and in which $R^4$ is phenyl unsubstituted or substituted by —$NH_2$, or is $C_1$-$C_{13}$ alkyl which is unsubstituted or substituted by phenyl, —$NH_2$, 2-oxopyrrolidino, cyano or one or two OH groups, and which can be interrupted by —O— or —S—, or in which $R^4$ is —$(CH_2)_m$—S—$CH_2$—CH(OH)—$CH_2$—S—($C_1$-$C_{16}$)-alkyl), m being zero to 4, or is —$(CH_2)_n$—CO—O—$R^5$, where $R^5$ is hydrogen, potassium or $C_4$-$C_{12}$-alkyl, and n is 1 or 2, or in which $R^4$ is —$(CH_2)_r$—CO—OH·$H_2N$—($C_{10}$-$C_{16}$-alkyl), r being 1 or 2, or is —P(S)—[O—$R^6$]$_2$, and $R^6$ is $C_1$-$C_8$ alkyl or phenyl, or in which $R^4$ is α-naphthyl, thiazolyl, benzothiazolyl, benzimidazolyl, benzoxazolyl, pyridyl, quinolyl or —$(CH_2)_s$—$R^7$ where s is 1 or 2 and $R^7$ is benzoxazolyl, benzimidazolyl, benzothiazolyl, thiazolinyl, imidazolinyl or oxazolinyl, or in which $R^4$ is —$(CH_2)_t$—CO—N($R^8$) ($R^9$), where t is 1 or 2, and $R^8$ is $C_1$-$C_4$ alkyl unsubstituted or substituted by —OH, or is phenyl or α-naphthyl, and $R^9$ is hydrogen or $R^8$, or in which $R^4$ is —$CH_2$—CH(OH)—$CH_2$—S—$R^{10}$, where $R^{10}$ is $C_1$-$C_{14}$ alkyl, or in which $R^4$ is a radical

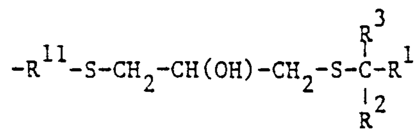

in which $R^1$, $R^2$ and $R^3$ have the meanings defined above, and $R^{11}$ is —$(CH_2)_2$—O—$(CH_2)_2$—O—$(CH_2)_2$—, o- or m-phenylene, thiadiazol-2,5-ylene or —$(CH_2)_u$—, u being zero to 4, preferably 2.

4. A composition according to claim 3, containing a lubricant or a hydraulic fluid and at least one compound of the formula II in which $R^1$, $R^2$ and $R^3$ together with the C atom to which they are bound are C-$C_{14}$ alkyl, and none of these substituents $R^1$, $R^2$ and $R^3$ may be hydrogen, and in which $R^4$ is phenyl, —$CH_2$—$CH_2$—$NH_2$, —$CH_2$—$CH_2$—OH, —$CH_2$—CH(OH)—$CH_2$—OH, tertiary $C_4$-$C_{14}$ alkyl, —$(CH_2)_2$—S—CH(OH)—$CH_2$—S—(tert-$C_8$-$C_{12}$ alkyl), —$CH_2$—COOH, —$CH_2$—CO—O—(i-$C_8H_{17}$), —$CH_2$—CO—OH·$H_2N$—(tert-$C_{10}$-$C_{16}$-alkyl), —P(S)—[O-(i-$C_3H_7$)]$_2$, —P(S)—[O—(i-$C_8H_{17}$)]$_2$, α-naphthyl, benzothiazolyl, benzimidazolyl, thiazolyl or —$CH_2$—CH(OH)—$CH_2$—S—$R^{10}$, where $R^{10}$ is tertiary $C_4$-$C_{14}$ alkyl, or in which $R^4$ is a radical

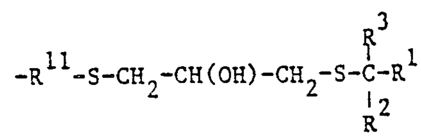

where $R^1$, $R^2$ and $R^3$ have the meanings defined above, and $R^{11}$ is —$(CH_2)_2$—O—$(CH_2)_2$—O—$(CH_2)_2$—, o-phenylene, thiadiazol-2,5-ylene or —$(CH_2)_u$—, u being zero to 2.

5. A compound of the formula II according to claim 2, in which $R^1$, $R^2$ and $R^3$ together with the C atom to which they are bound are $C_4$-$C_{20}$ alkyl, and none of these substituents $R^1$, $R^2$ and $R^3$ may be hydrogen, and in which $R^4$ is phenyl unsubstituted or substituted by —$NH_2$, or is unsubstituted $C_1$-$C_9$-alkyl, or $C_1$-$C_{16}$ alkyl which is substituted by phenyl, —$NH_2$, 2-oxo-pyrrolidino, cyano, perfluoro-$C_1$-$C_8$ alkyl or one or two OH groups, and which can be interrupted by —O— or —S—, or in which $R^4$ is —$(CH_2)_m$—S—$CH_2$—CH(OH)—($CH_2$—S—($C_1$-$C_{16}$-alkyl), m being zero to 6, or is —$(CH_2)_n$—C(O)—O—$R^5$, where n is 1 or 2, and $R^5$ is hydrogen, $C_1$-$C_{16}$-alkyl or an alkali metal, or in which $R^4$ is —CH[—CO—$OR^5$] [—$CH_2$—CO—$OR^5$], where $R^5$ has the meaning defined above, or in which $R^4$ is —$(CH_2)_r$—C(O)—OH·$H_2N$—($C_8$-$C_{16}$-alkyl) or —$(CH_2)_r$—C(O)—OH·N(—$CH_2$—$CH_2$—OH)$_3$, r being 1 or 2, or is —P(X)—[O—$R^6$]$_2$, where X can be = O or = S and $R^6$ is $C_1$-$C_{16}$-alkyl, phenyl or tolyl, or in which $R^4$ is α- or β-naphthyl, benzothiazolyl, benzimidazolyl, benzoxazolyl, thiazolyl, thiazolinyl, pyridyl, quinolyl, imidazolinyl, oxazolinyl, —$SO_2$—O—(alkali metal), —$C_6H_4$—C(O)—O—(alkali metal), 2-oxo-4-hydroxy-3-penten-3-yl or —$(CH_2)_s$—$R^7$, where s is 1 to 4, and $R^7$ is benzoxazolyl, benzimidazolyl, benzothiazolyl, thiazolinyl, imidazolinyl or oxazolinyl, or in which $R^4$ is —$(CH_2)_t$—CO—N($R^8$) ($R^9$), where t is 1 or 2, and $R^8$ is $C_1$-$C_{16}$-alkyl which is unsubstituted or substituted by —OH, or is phenyl, 3-hydroxyphenyl or α-naphthyl, and $R^9$ is hydrogen or $R^8$, or in which $R^4$ is —$CH_2$—CH(OH)—$CH_2$—S—$R^{10}$, where $R^{10}$ is hydrogen or $C_1$-$C_{16}$-alkyl or in which $R^4$ is a radical

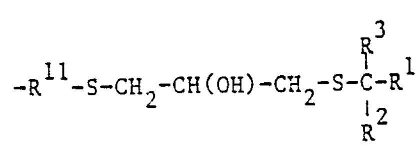

where $R^1$, $R^2$ and $R^3$ have the meanings defined above, and $R^{11}$ is a radical $-(CH_2)_2-O-(CH_2)_2-O-(CH_2)_2-$, o- or m-phenylene, thiadiazol-2,5-ylene or $-(CH_2)_u-$, u being zero to 8, or a radical of the formula

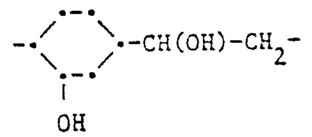 or

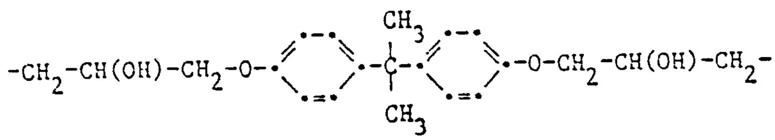

6. A compound according to claim 5 in which, in the formula II, $R^1$, $R^2$ and $R^3$ together with the C atom to which they are bound are $C_4$-$C_{20}$-alkyl, and none of these substituents $R^1$, $R^2$ and $R^3$ may be hydrogen, and in which $R^4$ is phenyl unsubstituted or substituted by $-NH_2$, unsubstituted $C_4$-$C_9$-alkyl, or $C_1$-$C_{13}$-alkyl which is substituted by phenyl, $-NH_2$, 2-oxopyrrolidino, cyano or one or two OH groups, or is $-(CH_2)_m-S-CH_2-CH(OH)-CH_2-S-(C_1$-$C_{16}$-alkyl), m being zero to 4, or is $-(CH_2)_n-CO-O-R^5$, wherein $R^5$ is hydrogen, potassium or $C_4$-$C_{12}$-alkyl, and n is 1 or 2, or in which $R^4$ is $-(CH_2)_r-CO-OH \cdot H_2N(C_{10}$-$C_{16}$-alkyl), r being 1 or 2, or is $-P(S)-[O-R^6]_2$, where $R^6$ is $C_1$-$C_8$-alkyl or phenyl, or in which $R^4$ is -naphthyl, thiazolyl, benzothiazolyl, benzimidazolyl, benzoxazolyl or $-(CH_2)_s-R^7$, where s is 1 or 2, and $R^7$ is benzoxazolyl, benzimidazolyl, benzothiazolyl, thiazolinyl, imidazolinyl or oxazolinyl, or in which $R^4$ is $-(CH_2)_t-CO-N(R^8)(R^9)$, where t is 1 or 2, and $R^8$ is $C_1$-$C_4$-alkyl which is unsubstituted or substituted by $-OH$, or is phenyl or α-naphthyl, and $R^9$ is hydrogen or $R^8$, or in which $R^4$ is $-CH_2-CH(OH)-CH_2-S-R^{10}$, where $R^{10}$ is $C_1$-$C_{14}$-alkyl, or in which $R^4$ is a radical

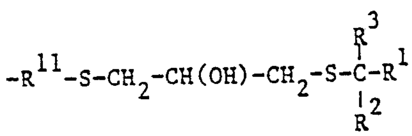

where $R^1$, $R^2$ and $R^3$ have the meanings defined above, and $R^{11}$ is $-(CH_2)_2-O-(CH_2)_2-O-(CH_2)_2-$, o- or m-phenylene, thiadiazol-2,5-ylene or $-(CH_2)_u-$, u being zero to 4, preferably 2.

7. A compound according to claim 6 in which, in the formula II, $R^1$, $R^2$ and $R^3$ together with the C atom to which they are bound are $C_4$-$C_{14}$-alkyl, and none of these substituents $R^1$, $R^2$ and $R^3$ may be hydrogen, and in which $R^4$ is phenyl, $-CH_2-CH_2-NH_2$, $-CH_2-CH_2-OH$, $-CH_2-CH(OH)-CH_2-OH$, tertiary $C_4$-$C_9$-alkyl, $-(CH_2)_2-S-CH(OH)-CH_2-S-$(tert-$C_8$-$C_{12}$-alkyl), $-CH_2COOH$, $-CH_2-CO-O-(i\text{-}C_8H_{17})$, $-CH_2-CO-OH \cdot H_2N-$(tert-$C_{10}$-$C_{16}$-alkyl), $-P(S)-[O-(i\text{-}C_3H_7)]_2$, $-P(S)-[P-(iC_8H_{17})]_2$, α-naphthyl, benzothiazolyl, benzimidazolyl, thiazolyl or $-CH_2-CH(OH)-CH_2-S-R^{10}$, where $R^{10}$ is tertiary $C_4$-$C_{14}$ alkyl, or in which $R^4$ is a radical

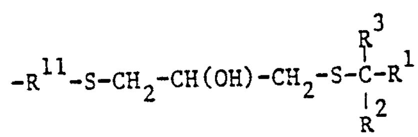

where $R^1$, $R^2$ and $R^3$ have the meanings defined above, and $R^{11}$ is $-(CH_2)_2-O-(CH_2)_2-O-(CH_2)_2-$, o-phenylene, thiadiazol-2,5-ylene or $-(CH_2)_u-$, u being zero to 2.

8. Use of a compound of the formula I according to claim 1 as an additive in lubricants.

## Revendications

1. Compositions qui contiennent un lubrifiant, ou un liquide hydraulique, et au moins composé répondant à la formule I :

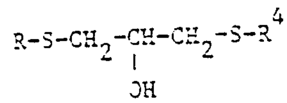 (I)

dans laquelle R représente un radical

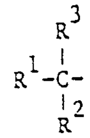

dans lequel $R^1$, $R^2$ et $R^3$ représentent chacun, indépendamment les uns des autres, un alkyle en $C_1$-$C_{18}$ et, ensemble, ne contiennent pas plus de 22 atomes de carbone, et $R^2$ et $R^3$ peuvent en outre représenter l'hydrogène, ou R représente un cycloalkyle à 5 ou 6 atomes de carbone, un radical phényle ou naphtyle non substitué ou porteur d'un alkyle en $C_1$-$C_4$, un benzyle, un furyle, un thiényle, un morpholinyle, un imidazolyle, un thiazolyle, un oxazolyle, un imidazolinyle, un thiazolinyle, un oxazolinyle, un benzimidazolinyle, un benzothiazolinyle ou un benzoxazolinyle, et $R^4$ représente un phényle non substitué ou porteur d'un $—NH_2$, un alkyle en $C_1$-$C_{16}$ non substitué ou porteur d'un phényle, d'un $—NH_2$, d'un oxo-2 pyrrolidino, d'un cyano, d'un perfluoro-alkyle en $C_1$-$C_8$ ou d'un ou de deux radicaux —OH, ce radical alkyle pouvant éventuellement être interrompu par —O— ou —S—, ou représente un cycloalkyle à 5 ou 6 atomes de carbone, ou $R^4$ représente un radical $—(CH_2)_m—S—CH_2—CH(OH)—CH_2—S—(C_1\text{-}C_{16}\text{-alkyl})$ dans lequel m désigne un nombre de 0 à 6, ou $R^4$ représente un radical $—(CH_2)_n—C(O)—O—R^5$ dans lequel n désigne le nombre 1 ou le nombre 2 et $R^5$ représente l'hydrogène, un alkyle en $C_1$-$C_{16}$ ou un métal alcalin, ou $R^4$ représente un radical $—CH\ [—CO—OR^5]\ [—CH_2—CO—OR^5]$ dans lequel $R^5$ a la signification qui lui a été donnée plus haut ou $R^4$ représente un radical $—(CH_2)_r—C(O)—OH \cdot H_2N—(C_8\text{-}C_{16}\text{-alkyl})$ ou un radical $—(CH_2)_r—C(O)—OH \cdot N—(CH_2—CH_2—OH)_3$ dans lesquels r est égal à 1 ou à 2, ou représente un radical $—P(X)—[O—R^6]_2$ dans lequel X représente O ou S et $R^6$ représente un alkyle en $C_1$-$C_{16}$, un phényle ou un tolyle, ou $R^4$ représente un radical α-naphtyle, β-naphtyle, benzothiazolyle, benzimidazolyle, benzoxazolyle, thiazolyle, thiazolinyle, triazolyle, tétrazolyle, pyridyle, quinolyle, imidazolyle, imidazolinyle, oxazolinyle, $—SO_2—O—$(métal alcalin), $—C_6H_4—C(O)—O—$(métal alcalin) ou oxo-2 hydroxy-4 pentène-3 yle-3 ou un radical $—(CH_2)_s—R^7$ dans lequel s désigne un nombre de 1 à 4 et $R^7$ représente un radical benzoxazolyle, benzimidazolyle, benzothiazolyle, thiazolinyle, imidazolinyle ou oxazolinyle, ou $R^4$ représente un radical $—(CH_2)_t—CO—N(R^8)\ (R^9)$ dans lequel t est égal à 1 ou à 2, $R^8$ représente un alkyle en $C_1$-$C_{16}$, éventuellement porteur d'un —OH, un phényle, un hydroxy-3 phényle ou un α-naphtyle, et $R^9$ représente l'hydrogène ou a la signification de $R^8$, ou $R^4$ représente un radical $—CH_2—CH(OH)—CH_2—S—R^{10}$ dans lequel $R^{10}$ représente l'hydrogène ou un alkyle en $C_1$-$C_{16}$, ou $R^4$ représente un radical $—R^{11}—S—CH_2—CH(OH)—CH_2—S—R$ dans lequel R a la signification qui lui a été donnée plus haut et $R^{11}$ représente un radical $—(CH_2)_2—O—(CH_2)_2—O—(CH_2)_2—$, o- ou m-phénylène ou thiadiazolylène-2,5, un radical $—(CH_2)_u—$ dans lequel u désigne un nombre de 0 à 8, ou un radical de formule :

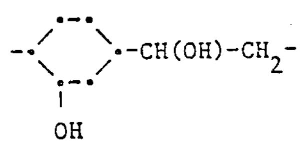

ou de formule :

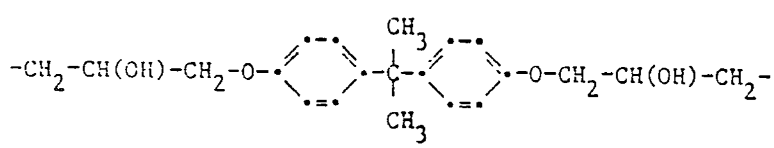

2. Compositions selon la revendication 1 qui contiennent un lubrifiant, ou un liquide hydraulique, et au moins un composé répondant à la formule II :

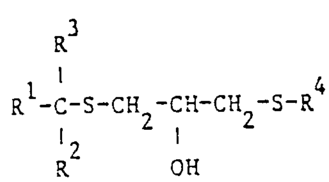

(II)

dans laquelle $R^1$, $R^2$ et $R^3$ forment ensemble, et avec l'atome de carbone auquel ils sont liés, un alkyle en $C_4$-$C_{20}$, et aucun de ces symboles $R^1$, $R^2$ et $R^3$ ne peut représenter l'hydrogène, et $R^4$ représente un phényle non substitué ou porteur d'un —$NH_2$, un alkyle en $C_1$-$C_{16}$ non substitué ou porteur d'un phényle, d'un —$NH_2$, d'un oxo-2 pyrrolidino, d'un cyano, d'un perfluoro-alkyle en $C_1$-$C_8$ ou d'un alkyle en $C_1$-$C_{16}$ lui-même porteur d'une ou de deux —OH, cet alkyle en $C_1$-$C_{16}$ pouvant éventuellement être interrompu par —O— ou —S—, ou représente un cycloalkyle à 5 ou 6 atomes de carbone, ou $R^4$ représente un radical —$(CH_2)_m$—S—$CH_2$—CH(OH)—$CH_2$—S—($C_1$-$C_{16}$-alkyl) dans lequel m désigne un nombre de 0 à 6, ou un radical —$(CH_2)_n$—C(O)—O—$R^5$ dans lequel n est égal à 1 ou à 2 et $R^5$ représente l'hydrogène, un alkyle en $C_1$-$C_{16}$ ou un métal alcalin, ou $R^4$ représente un radical —CH [—CO—$OR^5$] [—$CH_2$—CO—$OR^5$] dans lequel $R^5$ a la signification qui lui a été donnée ci-dessus, ou $R^4$ représente un radical répondant à l'une des formules —$(CH_2)_r$—C(O)—OH·$H_2N$—($C_8$-$C_{16}$-alkyl) et —$(CH_2)_r$—C(O)—OH·N($CH_2$—$CH_2$—OH$)_3$ dans lesquelles r est égal à 1 ou à 2, ou un radical —P(X)—[O—$R^6]_2$ dans lequel X représente O ou S et $R^6$ représente un alkyle en $C_1$-$C_{16}$, un phényle ou un tolyle, ou $R^4$ représente un radical α-naphtyle, β-naphtyle, benzothiazolyle, benzimidazolyle, benzoxazolyle, thiazolyle, thiazolinyle, pyridyle, quinolyle, imidazolinyle, oxazolinyle, —$SO_2$—O—(métal alcalin), —$C_6H_4$—C(O)—O—(métal alcalin), ou oxo-2 hydroxy-4 pentène-3 yle-3 ou un radical —$(CH_2)_s$—R dans lequel s désigne un nombre de 1 à 4 et $R^7$ représente un radical benzoxazolyle, benzimidazolyle, benzothiazolyle, thiazolinyle, imidazolinyle ou oxazolinyle, ou $R^4$ représente un radical —$(CH_2)_t$—CO—N($R^8$) ($R^9$) dans lequel t désigne un nombre égal à 1 ou à 2, $R^8$ représente un alkyle en $C_1$-$C_{16}$, éventuellement porteur d'un —OH, un phényle, un hydroxy-3 phényle ou un α-naphtyle, et $R^9$ représente l'hydrogène ou a la signification de $R^8$, ou $R^4$ représente un radical —$CH_2$—CH(OH)—$CH_2$—S—$R^{10}$ dans lequel $R^{10}$ représente l'hydrogène ou un alkyle en $C_1$-$C_{16}$, ou $R^4$ représente un radical :

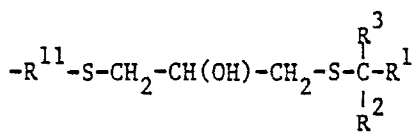

dans lequel $R^1$, $R^2$ et $R^3$ ont les significations qui leur ont été données plus haut, et $R^{11}$ représente un radical —$(CH_2)_2$—O—$(CH_2)_2$—O—$(CH_2)_2$—, o-phénylène, m-phénylène ou thiadiazolylène-2,5, un radical —$(CH_2)_u$— dans lequel u désigne un nombre de 0 à 8, de préférence le nombre 2, ou un radical répondant à l'une des formules :

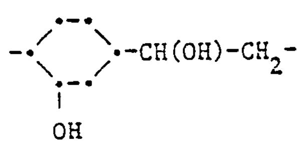

et

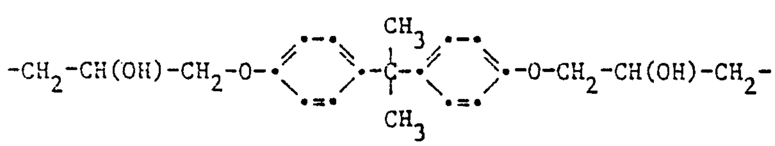

3. Compositions selon la revendication 2 qui contiennent un lubrifiant, ou un liquide hydraulique, et au moins un composé de formule II dans lequel $R^1$, $R^2$ et $R^3$ représentent ensemble, et avec l'atome de carbone auquel ils sont liés, un alkyle en $C_4$-$C_{20}$ et aucun de ces symboles R1, $R^2$ et $R^3$ ne peut représenter l'hydrogène, et $R^4$ représente un phényle non substitué ou porteur d'un —$NH_2$, ou un alkyle en $C_1$-$C_{13}$ non substitué ou porteur d'un phényle, d'un —$NH_2$, d'un oxo-2 pyrrolidino, d'un cyano ou d'un ou de deux —OH, cet alkyle pouvant éventuellement être interrompu par —O— ou —S—, ou $R^4$ représente un radical —$(CH_2)_m$—S—$CH_2$—CH(OH)—$CH_2$—S—($C_1$-$C_{16}$-alkyl) dans lequel m désigne un nombre de 0 à 4, ou un radical —$(CH_2)_n$—CO—O—$R^5$ dans lequel $R^5$ représente l'hydrogène, le potassium ou un alkyle en $C_4$-$C_{12}$ et n est égal à 1 ou à 2, ou $R^4$ représente un radical —$(CH_2)_r$—CO—OH·$H_2N$—($C_{10}$-$C_{16}$-alkyl) dans lequel r est égal à 1 ou à 2, ou un radical —P(S)—[O—$R^6]_2$ dans lequel $R^6$ représente un alkyle en $C_1$-$C_8$ ou un phényle, ou $R^4$ représente un radical α-naphtyle, thiazolyle, benzothiazolyle, benzimidazolyle, benzoxazolyle, pyridyle ou quinolyle, ou un radical —$(CH_2)_s$—$R^7$ dans lequel s est égal à 1 ou à 2 et $R^7$ représente un radical benzoxazolyle, benzimidazolyle, benzothiazolyle, thiazolinyle, imidazolinyle ou oxazolinyle, ou $R^4$ représente un radical —$(CH_2)_t$—CO—N($R^8$) ($R^9$) dans lequel t est égal à 1 ou à 2, $R^8$ représente un alkyle en $C_1$-$C_4$ éventuellement porteur d'un —OH, un phényle ou un α-naphtyle, et $R^9$ représente l'hydrogène ou a la signification qui a été donnée pour $R^8$, ou $R^4$ représente un radical —$CH_2$—CH(OH)—$CH_2$—S—$R^{10}$ dans lequel $R^{10}$ représente un alkyle en $C_1$-$C_{14}$, ou $R^4$ représente un radical :

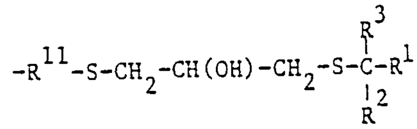

dans lequel $R^1$, $R^2$ et $R^3$ ont les significations qui leur ont été données ci-dessus et $R^{11}$ représente un radical $—(CH_2)_2—O—(CH_2)_2—O—(CH_2)_2—$, o-phénylène, m-phénylène ou thiadiazolylène-2,5 ou un radical $—(CH_2)_u—$ dans lequel u désigne un nombre de 0 à 4, de préférence le nombre 2.

4. Compositions selon la revendication 3 qui contiennent un lubrifiant, ou un liquide hydraulique, et au moins un composé de formule II dans lequel $R^1$, $R^2$ et $R^3$ représentent ensemble, et avec l'atome de carbone auquel ils sont liés, un alkyle en $C_4$-$C_{14}$, et aucun de ces symboles $R^1$, $R^2$ et $R^3$ ne peut représenter l'hydrogène, et $R^4$ représente un radical phényle, $—CH_2—CH_2—NH_2$, $—CH_2—CH_2—OH$, $—CH_2—CH(OH)—CH_2—OH$, tert-alkyle en $C_4$-$C_{14}$, $—(CH_2)_2—S—CH(OH)—CH_2—S—$(tert-$C_8$-$C_{12}$-alkyl), $—CH_2—COOH$, $—CH_2—CO—O—$(i-$C_8H_{17}$), $—CH_2—CO—OH·H_2N—$(tert-$C_{10}$-$C_{16}$-alkyl), $—P(S)$ [O—(i-$C_3H_7$)]$_2$, $—P(S)$ [O—(i-$C_8H_{17}$)]$_2$, α-naphtyle, benzothiazolyle, benzimidazolyle, thiazolyle ou $—CH_2—CH(OH)—CH_2—S—R^{10}$, le symbole $R^{10}$ désignant un alkyle tertiaire en $C_4$-$C_{14}$, ou $R^4$ représente un radical :

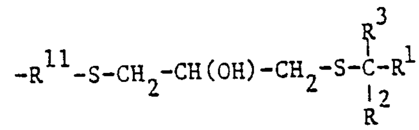

dans lequel $R^1$, $R^2$ et $R^3$ ont les significations qui leur ont été données plus haut et $R^{11}$ représente un radical $—(CH_2)_2—O—(CH_2)_2—O—(CH_2)_2—$, o-phénylène ou thiadiazolylène-2,5 ou un radical $—(CH_2)_u—$ dans lequel u désigne un nombre de 0 à 2.

5. Composés de formule II selon la revendication 2 dans lesquels $R^1$, $R^2$ et $R^3$ représentent ensemble, et avec l'atome de carbone auquel ils sont liés, un alkyle en $C_4$-$C_{20}$, et aucun de ces symboles $R^1$, $R^2$ et $R^3$ ne peut représenter l'hydrogène, et $R^4$ représente un phényle non substitué ou porteur d'un $—NH_2$, un alkyle en $C_1$-$C_9$ non substitué ou un alkyle en $C_1$-$C_{16}$ porteur d'un phényle, d'un $—NH_2$, d'un oxo-2 pyrrolidino, d'un cyano, d'un perfluoro-alkyle en $C_1$-$C_8$ et d'un ou de deux —OH, alkyle qui peut éventuellement être interrompu par —O— ou —S—, ou $R^4$ représente un radical $—(CH_2)_m—S—CH_2—CH(OH)—CH_2—S—$($C_1$-$C_{16}$-alkyl) dans lequel m désigne un nombre de 0 à 6, ou un radical $—(CH_2)_n—C(O)—O—R^5$ dans lequel n est égal à 1 ou à 2 et $R^5$ représente l'hydrogène, un alkyle en $C_1$-$C_{16}$ ou un métal alcalin, ou $R^4$ représente un radical $—CH[—CO—OR^5]$ $[—CH_2—CO—OR^5]$ dans lequel $R^5$ a la signification qui lui a été précédemment donnée, ou $R^4$ représente un radical $—(CH_2)_r—C(O)—OH·H_2N$($C_8$-$C_{16}$-alkyl) ou un radical $—(CH_2)_r—C(O)—OH·N(—CH_2—CH_2—OH)_3$ dans lesquels r est égal à 1 ou à 2, ou un radical $—P(X)$ $[O—R^6]_2$ dans lequel X représente O ou S et $R^6$ représente un alkyle en $C_1$-$C_{16}$, un phényle ou un tolyle, ou $R^4$ représente un radical α-naphtyle, β-naphtyle, benzothiazolyle, benzimidazolyle, benzoxazolyle, thiazolyle, thiazolinyle, pyridyle, quinolyle, imidazolinyle, oxazolinyle, $—SO_2—O—$(métal alcalin), $—C_6H_4—C(O)—O—$(métal alcalin) ou oxo-2 hydroxy-4 pentène-3 yle-3 ou un radical $—(CH_2)_s—R^7$ dans lequel s désigne un nombre de 1 à 4 et $R^7$ représente un radical benzoxazolyle, benzimidazolyle, benzothiazolyle, thiazolinyle, imidazolinyle ou oxazolinyle, ou $R^4$ représente un radical $—(CH_2)_t—CO—N(R^8)$ $(R^9)$ dans lequel t est égal à 1 ou à 2, $R^8$ représente un alkyle en $C_1$-$C_{16}$, éventuellement porteur d'un —OH, un phényle, un hydroxy-3 phényle ou un α-naphtyle, et $R^9$ représente l'hydrogène ou a la signification de $R^8$, ou $R^4$ représente un radical $—CH_2—CH(OH)—CH_2—S—R^{10}$ dans lequel $R^{10}$ représente l'hydrogène ou un alkyle en $C_1$-$C_{16}$, ou $R_4$ représente un radical :

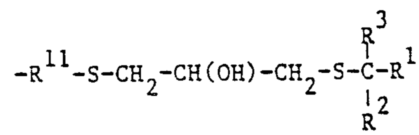

dans lequel $R^1$, $R^2$ et $R^3$ ont les significations qui leur ont été données ci-dessus et $R^{11}$ représente un radical $—(CH_2)_2—O—(CH_2)_2—O—(CH_2)_2—$, o-phénylène, m-phénylène ou thiadiazolylène-2,5, un radical $—(CH_2)_u—$ dans lequel u désigne un nombre de 0 à 8, ou un radical répondant à l'une des formules :

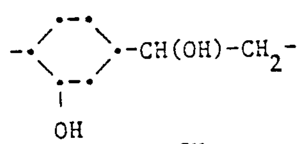 et

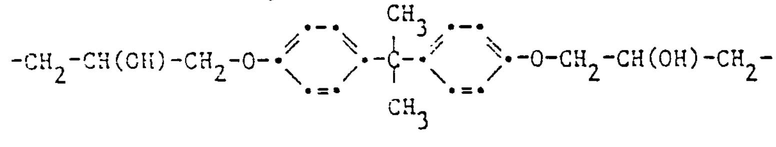

6. Composés selon la revendication 5 dans la formule II desquels $R^1$, $R^2$ et $R^3$ représentent ensemble, et avec l'atome de carbone auquel ils sont liés, un alkyle en $C_4$-$C_{20}$, et aucun de ces symboles $R^1$, $R^2$ et $R^3$ ne peut représenter l'hydrogène, et dans lesquels $R^4$ représente un phényle non substitué ou porteur d'un $—NH_2$, un alkyle en $C_4$-$C_9$ non substitué, un alkyle en $C_1$-$C_{13}$ porteur d'un phényle, d'un $—NH_2$, d'un oxo-2 pyrrolidino, d'un cyano ou d'un ou de deux —OH, un radical $—(CH_2)_m—S—CH_2—CH(OH)—CH_2—S—(C_1\text{-}C_{16}\text{-alkyl})$ dans lequel m désigne un nombre de 0 à 4, ou un radical $—(CH_2)_n—CO—O—R^5$ dans lequel $R^5$ représente l'hydrogène, le potassium ou un alkyle en $C_4$-$C_{12}$ et n est égal à 1 ou à 2, ou $R^4$ représente un radical $—(CH_2)_r—CO—OH{\cdot}H_2N(C_{10}\text{-}C_{16}\text{-alkyl})$ dans lequel r est égal à 1 ou à 2, ou un radical $—P(S)\ [O—R^6]_2$ dans lequel $R^6$ représente un alkyle en $C_1$-$C_8$ ou un phényle, ou $R^4$ représente un radical α-naphtyle, thiazolyle, benzothiazolyle, benzimidazolyle ou benzoxazolyle ou un radical $—(CH_2)_s—R^7$ dans lequel s est égal à 1 ou à 2 et $R^7$ représente un radical benzoxazolyle, benzimidazolyle, benzothiazolyle, thiazolinyle, imidazolinyle ou oxazolinyle, ou $R^4$ représente un radical $—(CH_2)_t—CO—N(R^8)\ (R^9)$ dans lequel t est égal à 1 ou à 2, $R^8$ représente un alkyle en $C_1$-$C_4$, éventuellement porteur d'un —OH, un phényle ou un α-naphtyle, et $R^9$ représente l'hydrogène ou a la signification de $R^8$, ou $R^4$ représente un radical $—CH_2—CH(OH)—CH_2—S—R^{10}$ dans lequel $R^{10}$ représente un alkyle en $C_1$-$C_{14}$, ou représente un radical :

$$-R^{11}-S-CH_2-CH(OH)-CH_2-S-\overset{R^3}{\underset{R^2}{C}}-R^1$$

dans lequel $R^1$, $R^2$ et $R^3$ ont les significations qui leur ont été données ci-dessus et $R^{11}$ représente un radical $—(CH_2)_2—O—(CH_2)_2—O—(CH_2)_2—$, o-phénylène, m-phénylène ou thiadiazolylène-2,5 ou un radical $—(CH_2)_u—$ dans lequel u désigne un nombre de 0 à 4, de préférence le nombre 2.

7. Composés selon la revendication 2 dans la formule II desquels $R^1$, $R^2$ et $R^3$ représentent ensemble, et avec l'atome de carbone auquel ils sont liés, un alkyle en $C_4$-$C_{14}$, et aucun de ces symboles $R^1$, $R^2$ et $R^3$ ne peut représenter l'hydrogène, et $R^4$ représente un radical phényle, $—CH_2—CH_2—NH_2$, $—CH_2—CH_2—OH$, $—CH_2—CH(OH)—CH_2—OH$, tert-alkyle en $C_4$-$C_9$, $—(CH_2)_2—S—CH(OH)—CH_2—S—(\text{tert-}C_8\text{-}C_{12}\text{-alkyl})$, $—CH_2COOH$, $—CH_2—CO—O—(i—C_8H_{17})$, $—CH_2—CO—OH—H_2N—(\text{tert-}C_{10}\text{-}C_{16}\text{-alkyl})$, $—P(S)\ [—O—(i\text{-}C_3H_7)]_2$, $—P(S)\ [—O—(i\text{-}C_8H_{17})]_2$, α-naphtyle, benzothiazolyle, benzimidazolyle ou thiazolyle ou un radical $—CH_2—CH(OH)—CH_2—S—R^{10}$ dans lequel $R^{10}$ représente un alkyle tertiaire en $C_4$-$C_{14}$, où $R^4$ représente un radical :

$$-R^{11}-S-CH_2-CH(OH)-CH_2-S-\overset{R^3}{\underset{R^2}{C}}-R^1$$

dans lequel $R^1$, $R^2$ et $R^3$ ont les significations qui leur ont éte données ci-dessus et $R^{11}$ représente un radical $—(CH_2)_2—O—(CH_2)_2—O—(CH_2)_2—$, o-phénylène ou thiadiazolylène-2,5 ou un radical $—(CH_2)_u—$ dont l'indice u désigne un nombre de 0 à 2.

8. Application de composés de formule I selon la revendication 1 comme additifs dans des lubrifiants.